(19) **Europäisches Patentamt / European Patent Office / Office européen des brevets**

(11) **EP 2 730 926 B1**

(12) **FASCICULE DE BREVET EUROPEEN**

(45) Date de publication et mention
de la délivrance du brevet:
**19.12.2018 Bulletin 2018/51**

(51) Int Cl.:
*G01N 33/84* (2006.01)        *A01C 21/00* (2006.01)
*C12N 1/00* (2006.01)

(21) Numéro de dépôt: **13366006.8**

(22) Date de dépôt: **12.11.2013**

(54) **Diagnostique de l'état microbiologique de sols en fonction de la resilience de populations bactériennes qu'ils contiennent**

Diagnose des mikrobiologischen Zustands des Bodens anhand der Widerstandsfähigkeit der darin vorhandenen Bakterienpopulationen

Diagnosis of the microbiological state of soils according to the resilience of its bacterial population.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.11.2012 FR 1203051**

(43) Date de publication de la demande:
**14.05.2014 Bulletin 2014/20**

(73) Titulaire: **Polyor SARL**
**54000 Nancy (FR)**

(72) Inventeur: **CLAUDE, Pierre-Philippe**
**54000 Nancy (FR)**

(56) Documents cités:
**EP-A1- 2 409 561        US-A1- 2012 014 748**

- **W.A. DICK ET AL: "Soil acid and alkaline phosphatase activity as pH adjustment indicators", SOIL BIOLOGY AND BIOCHEMISTRY, vol. 32, no. 13, 1 novembre 2000 (2000-11-01), pages 1915-1919, XP055073613, ISSN: 0038-0717, DOI: 10.1016/S0038-0717(00)00166-8**
- **DAVID FERNÁNDEZ-CALVIÑO ET AL: "Growth response of the bacterial community to pH in soils differing in pH", FEMS MICROBIOLOGY ECOLOGY, 1 avril 2010 (2010-04-01), pages no-no, XP055053363, ISSN: 0168-6496, DOI: 10.1111/j.1574-6941.2010.00873.x**
- **JOHANNES ROUSK ET AL: "Soil bacterial and fungal communities across a pH gradient in an arable soil", THE ISME JOURNAL, vol. 4, no. 10, 6 mai 2010 (2010-05-06), pages 1340-1351, XP055053758, ISSN: 1751-7362, DOI: 10.1038/ismej.2010.58**

EP 2 730 926 B1

**Description**

**DOMAINE TECHNIQUE DE L'INVENTION**

Analyses biologique et/ou physico-chimique des sols arables, et fertilisation des cultures agronomiques. Accessoirement, il est aussi question de microbiologie des sols et de biofertilisation par inoculation azotobactérienne.

**ÉTAT DE LA TECHNIQUE**

***La biofertilisation bactérienne des rhizo - et résidu - sphères***

Les BFCP (bactéries favorisant la croissance des plantes) colonisent les racines de certaines cultures, les résidus de cultures cellulosiques, les engrais organominéraux et/ou les biomasses racinaires résiduelles. Les BFCP appartiennent à plusieurs genres, y compris *Agrobacterium, Alcaligenes, Arthrobacter, Azotobacter, Bacillus, Cellulomonas, Flavobacterium, Pseudomonas, (Brady)rhizobium* et *Xanthomonas.* Leurs modes d'action comprennent : (i) la production de sidérophores extracellulaires (agents microbiens de transport du fer) qui peuvent s'associer de manière efficace avec le fer présent dans l'environnement en le rendant moins disponible pour certaines microflores naturelles non-phytogènes, (ii) l'antibiose contre des bactéries et des champignons pathogènes, (iii) la production de substances favorisant la croissance, et iv) la solubilisation des phosphates organiques et inorganiques. Plus particulièrement, la biofertilisation azotobactérienne consiste en l'utilisation d'inocula BFCP diazotrophes, avantageusement *Azotobaceraceae.* Les *Azotobacteraceae* sont des bactéries libres, aérobies et capables de fixer l'azote de manière non symbiotique. Leurs populations peuvent varier dans le sol mais ne dépasse que très rarement $10^3$ - $10^4$ / g de sol (Roper *et al.* 1995).

Les bactéries réintroduites dans la *rhizosphère* par inoculation des semences accroîtra exsudation, du carbone par les racines (Jones *et al.* 2004), et cela au risque de ponctionner le flux photosynthétique au dépend des rendements agronomiques (Lynch et Ho 2005). *In contrario,* la bactérisation des *résidusphères* par inoculation des résidus de culture au sol, en procurant aux dites BFCP une source alternative de carbone, évitera cette ponction dudit flux photosynthétique (Claude et Fillion 2004, Claude et Giroux 2006, Halsall et Gibson 1991). Cela dit, l'acidité du sol (pH) affectera beaucoup plus l'activité, la taille et la composition génomique de telles populations bactériennes.

***L'acidité du sol et la biofertilisation en sols arables***

Les trois (3) groupes d'acidités dans les sols s'additionnent pour donner l'acidité totale, à savoir ;

- L'acidité active est due aux ions H+ dans la solution (eau) du sol. Dans les faits, l'acidité active est très petite comparativement aux acidités échangeable et résiduelle, mais très importante puisqu'elle détermine la solubilité de plusieurs éléments et affect la qualité du milieu dans lequel baigne les racines et microorganismes.

- L'acidité échangeable est elle attribuable à l'aluminium et l'hydrogène adsorbés sur le complexe d'échange du sol (*cf.* CEC); elle est échangeables par d'autres cations tels que le K⁺ Le $pH_{KCl}$ permet donc de mesurer grossièrement l'acidité d'échange qui nous intéresse particulièrement ici. En effet, lorsque le sol est ainsi mélangé à une solution de KCl, voire de $CaCl_2$, ces cations vont chasser les H⁺ et $Al^{3+}$fixés sur le complexe d'échange ; ce $pH_{KCl}$ est généralement inférieur au pH eau de 0,2 à 1,5, voire 2,0 unités pH (dpH). L'acidité échangeable peut aussi être mesuré plus précisément par titrage acido-basique de l'acidité non - résiduelle.

- L'acidité résiduelle peut elle être neutralisée par des matériaux alcalins tels que la chaux, mais ne peut être détectée à l'aide d'un sel remplaçable (*eg.* KCl). Elle est associée avec les ions Al et H, y compris les ions hydroxydes d'aluminium, liés par la matière organique et les argiles. L'acidité résiduelle est parfois 1 000 fois supérieure à l'acidité active ou échangeable dans un sol sablonneux, voire plus de 50 000 fois supérieure dans un sol argileux riche en matière organique.

Les variations de pH au cour d'une campagne (saison) seront donc nécessairement le plus souvent encadrées par dpH, mesure approximative de l'acidité échangeable d'un sol arable. Ces variations de pH intra - saisonnières seront conséquentes pour la biofertilisation bactérienne, y compris par azotobactérisation des résidus de culture au sol (*eg.* Claude et Fillion 2004). En effet, l'apport d'aussi petites quantités de biomasses bactériennes ne représentant que 20 des quelques 500 000 g de biomasses bactériennes à l'hectare peut-elle avoir un effet sur la dynamique de la fixation de l'azote dans la résidusphère ? Il faut se rappeler que la résidusphère n'occupe que 1 à 3% du volume du sol sur 10 cm de profondeur; la résidusphère n'est donc en présence que 5 à 15 kg de biomasse bactérienne indigène. De plus, les *Azotobacteraceae* indigènes sont relativement peu nombreuses, soit de l'ordre de $10^3$ à $10^5$ cellules par g sur un total de l'ordre de $10^9$ cellules bactériennes ; les 20 g de cellules *Azotobacteraceae* inoculées sont donc pondéralement comparables aux *Azotobacteraceae* indigènes affectant directement la résidusphère. Donc, étant donnée cette suffisance, pourquoi inoculer à l'aide de produits de type Solactiv™ ? Comment savoir s'il est nécessaire, ou du moins utile de renforcer ces populations par inoculation ?

Il faut donc savoir dans quelle mesure la dynamique diazotrophe des *Azotobacteraceae résiste* aux variations de pH du sol normalement attendues à travers une campagne, i.e. dans le cadre de l'acidité échangeable mesurés. Cette acidité échangeable est la différence entre les mesures du pH du sol dans des solutions d'extraction aqueuses et salines (Kissel *et al.* 2009 ; Miller et Kissel 2010). En effet, le dpH déterminé par mesure électrométrique de solutions d'extraction échantillons de KCl (*cf.* norme ISO 10390) est le plus souvent de 0,5 à 1,5 unités puisque les ions $K^+$ s'échangent avec des ions $H_3O^+$ qui n'étaient pas dissociées en solution aqueuse. Pourtant, à ce jour, le diagnostique de l'état microbiologique de sols arables ne permet pas de mesurer cette résilience des populations bactériennes, et plus particulièrement *Azotobacteraceae,* du sol confrontées à des variations de pH somme toutes normales.

### *Le diagnostique microbiologique des sols arables*

Le diagnostique de l'état microbiologique de sols arables fait aujourd'hui l'objet de maintes recherches, voire occasionnellement d'offre commerciales. Pourtant, à ce jour ce type de diagnostique est subjectif, voire au mieux qualitatif ;

- SIR (*substrat induced respiration* ; Anderson et Domsch 1978)
- CLPP (*community-level physiologicalprofile* ; Campbell *et al.* 2003)
- PFLA (*phospholipid fatty acid*; Rousk *et al.* 2010)
- T/DGGE (*temperature / density gradient gel electrophoresis* ; Wakelin *et al.* 2008)
- PCR-Q (PCR quantitative; Picard *et al.* 1992
- MDM (*microbial diagnostic microarrays*; Sessitsch *et al.* 2006)
- *Fungal / Bacterial ratios* (Lauber *et al.* 2008)
- "Cycle activity indicator" (Kubota 2012; US 2012/0014748 A1)

A l'exception peut-être de Kubota 2012, ces approches sont surtout descriptives, et mettent simplement en évidence des différences, souvent banales, entre différents types de sols, *eg.* prairies versus sols cultivés versus sols forestiers). Kubota 2012 est lui plus pragmatique. Il conçoit un indice visuel sous la forme d'un triangle dont l'aire représente en sorte le niveau d'activité microbienne du sol, ou encore d'une proportion chiffrée équivalente à ladite aire. En terme de N, P et K, ladite aire est donc celle d'un triangle dont les pointes sont numériquement le nombre de cellules bactériennes dans le sol, et selon le cas (N, P ou K, respectivement), i) le taux de disparition de l'$NH_4$ et/ou du $NO_2$, ii) la formation de $PO_4$ à partir de phytine et/ou de compostes, et enfin iii) le re-largage de K d'un sol de référence et/ou de compostes. Outre le fait que le choix de ces variables indicatrices me semble peu fondé, il n'est ici nullement question de pH optima pour l'activité des populations bactériennes du sol.

Il est impossible à ce jour de diagnostiquer si les populations bactériennes sont oui ou non adaptées au pH du sol actuel et/ou s'il est avantageux de modifier le pH afin d'avantager la flore bactérienne, ou encore de renforcer par inoculation certaines composantes de cette flore bactérienne. La notion même de *pH optimal* (pHo) du sol fait débat (*eg.* Simek *et al.* 2002), surtout d'un pont de vue microbiologique. En effet, bon nombre d'études tentent de relier pH du sol et certaines mesures d'activité, de diversité et/ou d'abondance (relative et absolue) des populations bactériennes. Il y a bel et bien un-rôle important, voire déterminant, du pH du sol puisque ces dynamiques sont décrites par des courbes polynomiales concaves affichant des pH optimaux (pHo ; Simek et Hopkins 1999, Fernandez-Calvino et Bääth 2010, Rosso *et al.* 1995, etc. ; Tableau 1).

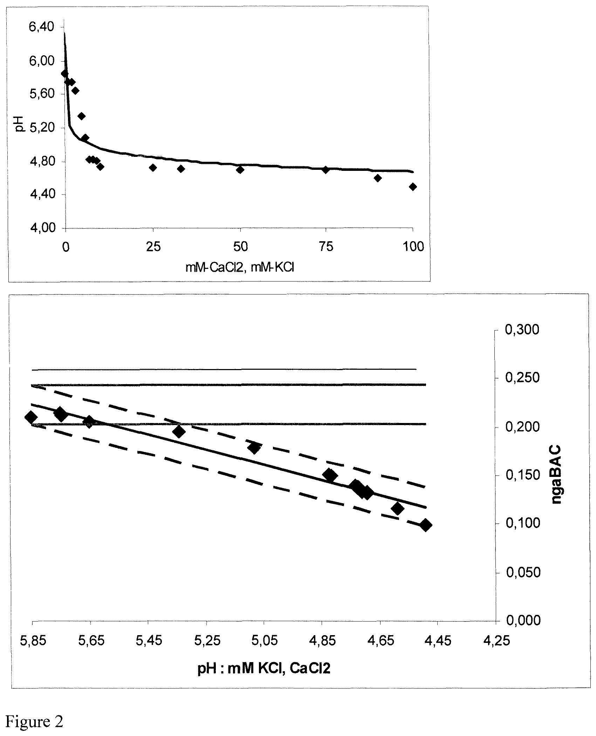

**Tableau 1** : Dynamique métabolique, massique ou génomique (nga) de la fraction bactérienne du sol (BAC) pour le calcul de la première dérivée d'une fonction polynomiales de troisième degré au pHo (f(pHo) = 0,00). Les coefficients a, b, c et d de ces fonctions polynomiales de troisième degré sont rapportés au Tableau 2. Les études en **gras** servirent ici d'exemples d'applications (Figures 2, 3 et 4 ; Tableau 4)

| Référence | nombre, masse **(g)**, activité (nga) **BAC** | pHo | courbure[1] |
|---|---|---|---|
| Jones *et al.* 2009 (Figure 2-1) | abondance relative | 4,29 | -0,51 |
| Pankratov *et al.* 2008 (Figure 5) | u (/hr) in vitro | 4,52 | -0,54 |
| Peterse *et al.* 2010 Figure 2 | CBT (bactériens) | 4,75 | -0,59 |

(suite)

| Référence | nombre, masse (g), activité (nga) BAC | pHo | courbure[1] |
|---|---|---|---|
| Fernandez-Calvino et Baath 2010 (Fig. 2/4,5) | activité BAC (incorporation THY) | 4,78 | -0,50 |
| Bäath 1998 (Tableau 1) pH KCl | activité BAC (incorporation LEU) | 4,87 | -0,46 |
| Kemmitt *et al.* 2005 figure 5 | activité BAC assimilation Acaminé | 5,24 | -0,18 |
| Bäath 1998 (Tableau 1) pH KCl | activité BAC (incorporation THY) | 5,27 | -0,38 |
| Baath *et al.* 1992 Figure 2 (BAC1) | activité BAC (incorporation THY) | 5,36 | -0,24 |
| **Baath et al. 1995 Figure 6** | **activité BAC (incorporation THY)** | **5,36** | **-0,22** |
| Bäath 1998 (Tableau 1) pH eau | activité BAC (incorporation LEU) | 5,57 | -0,37 |
| Bäath **1998 (Tableau 1) pH eau** | **activité BAC (incorporation THY)** | **5,85** | **-0,41** |
| Fernandez-Calvino et Baath 2010 (Fig. 2/5,5) | activité BAC (incorporation THY) | 6,31 | -0,44 |
| Koga *et al.* 2003 (Tableau 3) | croissance MFT in situ | 6,37 | -0,46 |
| **Ownley *et al.* 1992 (Figure 2)** | **er Pseudomonas ilnhibition Pathos** | **6,38** | **-0,41** |
| Parkin *et al.* 1985 Figure 1 (limed) | activité dénitrification in situ | 6,40 | -0,50 |
| Fernandez-Calvino et Baath 2010 (Fig. 2/6,9) | activité BAC (incorporation THY) | 6,67 | -0,49 |
| Fernandez-Calvino et Baath 2010 (Fig. 2/6,5) | activité BAC (incorporation THY) | 6,76 | -0,60 |
| Rousk *et al.* 2010 Figure 4 c/n combo | abondance relative | 6,96 | -0,56 |
| Rousk *et al.* 2010 Figure 4 a/n combo | abondance relative | 7,03 | -0,71 |
| Rousk *et al.* 2010c Figure 1 (alfalfa) | activité BAC (incorporation LEU) | 7,17 | -0,35 |
| Rousk *et al.* 2010c Figure 1C | activité BAC (incorporation LEU) | 7,26 | -0,40 |
| Rousk *et al.* 2010c Figure 1 (paille) | activité BAC (incorporation LEU) | 7,31 | -0,48 |
| Taziebou *et al.* 2004 (Figture 1a) | nCFU | 7,42 | -0,44 |
| Rousk *et al.* 2009 Figure 2A | activité BAC (incorporation LEU) | 7,46 | -0,47 |
| Ownley *et al.* 1992 (Figure 4) | er Pseudomonas iPathos Pfa | 7,47 | -0,50 |
| Lauber *et al.* 2009 Figure 5 combo | abondance relative (%) | 7,47 | -0,40 |
| Simek et Hopkins 1999 Figure 3 | activité dénitrification in situ | 7,49 | -0,43 |
| Ownley *et al.* 1992 (Figure 4) | er Pseudomonas iPathos Pfa bis | 7,55 | -0,92 |
| Jones *et al.* 2009 (Figure 2-4) | abondance relative | 7,78 | -0,48 |
| Simek *et al.* 2002 Figure 4 | activité dénitrification in situ | 7,85 | -0,72 |
| Simek *et al.* 2002 Figure 7 | activité dénitrification in situ | 8,13 | -1,16 |

(suite)

| Référence | nombre, masse **(g)**, activité (nga) **BAC** | **pHo** | courbure[1] |
|---|---|---|---|
| Simek *et al.* 2002 Figure 5 | activité dénitrification in situ | 8,24 | -0,98 |
| Parkin *et al.* 1985 Figure 1 (unlimed) | activité dénitrification in situ | 8,39 | -0,99 |

**Note 1 :** *courbure relative*, ou ratio de [ngaBAC = f'(pH) | pHo] / [ngaBAC = f(pH) | pHo]. Il est ainsi possible de comparer le degré de courbure des fonctions [ngaBAC : pH] calculer à partir de données des différents auteurs, provenant de sols différents et utilisant des mesures de la dynamique ngaBAC variées.

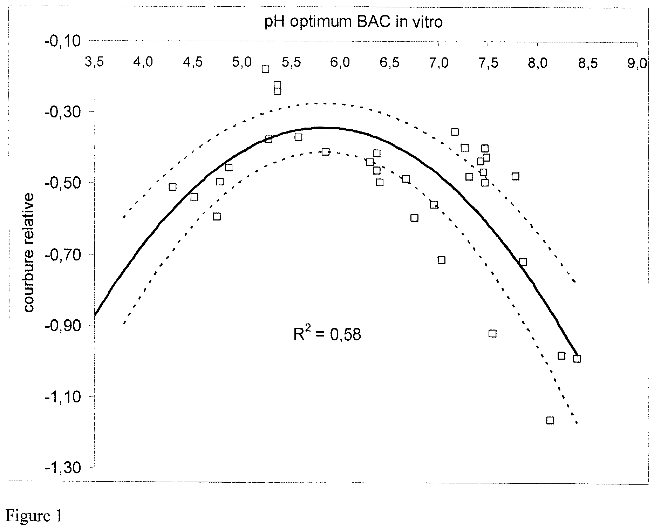

A partir d'études décrivant de telles dynamiques des populations bactériennes de sols en fonction du pH, j'ai donc calculé la seconde dérivée f''(pH) → courbure à pHo, *i.e.* lorsque la première dérivée, f'(pH) → pente égale 0,00. Les courbures des fonctions [ngaBAC : pH] (Tableau 1, Figure 1) sont les plus prononcées à pHo soit très acides ou très alcalins, les pHo plus près de 7, aux alentours de 6,00 à 6,50, affichent des courbures moins importantes caractéristiques de populations bactériennes plus tolérantes à des variations du pH environnant, *i.e.* plus *résilientes.* A noter que les pHo sont rarement les mêmes du fait de sols et/ou de populations bactériennes très différents. Il est ainsi possible en principe de s'affranchir de pHo comme mesure diagnostique en calculant plutôt la courbure de la fonction [ngaBAC : pH] à pHo (Figure 1). Ainsi, les populations bactériennes de sols plutôt acides devraient raisonnablement afficher des courbes [ngaBAC : pH] plus abruptes. En effet, de tels pH acides favoriseront la sélection de populations bactériennes pour lesquelles les conditions acido-basiques optimales sont plus étroitement définies.

**Tableau 2** : Coefficients des équations polynomiales $pH = aX^3 + bX^2 + cX + d$ pour chacun des jeux de données publiés au Tableau 1. En **gras**, les données ayant servi à générer les trois (3) cas de figures à titre d'exemples d'applications (*cf*. Tableau 3 et Figure 2, 3 et 4).

| Référence | **a** | **b** | **c** | **d** | pHo |
|---|---|---|---|---|---|
| Jones *et al.* 2009 (Figure 2-1) | 0,021 | -0,387 | 2,154 | -3,340 | 4,29 |
| Pankratov *et al.* 2008 (Figure 5) | 0,007 | -0,120 | 0,669 | -1,097 | 4,52 |
| Peterse *et al.* 2010 Figure 2 | 0,113 | -2,003 | 11,352 | -19,568 | 4,75 |
| Fernandez-Calvino et Baath 2010 (Fig. 2/4,5) | 0,069 | -1,302 | 7,717 | -13,412 | 4,78 |
| Bäath 1998 (Tableau 1) pH KCl | 0,019 | -0,334 | 1,909 | -3,303 | 4,87 |
| Kemmitt *et al.* 2005 figure 5 | -0,847 | 7,275 | -6,500 | 22,852 | 5,24 |
| Baath 1998 (Tableau 1) pH KCl | 0,009 | -0,178 | 1,136 | -2,144 | 5,27 |
| Baath *et al.* 1992 Figure 2 (BAC1) | 1,482 | -39,155 | 292,120 | -543,350 | 5,36 |
| **Baath et *al.* 1995 Figure 6** | **0,489** | **-16,482** | **134,650** | **-246,160** | 5,36 |
| Bäath 1998 (Tableau 1) pH eau | 0,018 | -0,345 | 2,161 | **-4,228** | 5,57 |
| **Bäath 1998 (Tableau 1) pH eau** | **0,013** | **-0,274** | **1,860** | **-3,912** | 5,85 |
| Fernandez-Calvino et Baath 2010 (Fig. 2/5,5) | -0,039 | 0,524 | -1,921 | 2,124 | 6,31 |
| Koga *et al.* 2003 (Tableau 3) | -0,022 | 0,274 | -0,788 | 0,290 | 6,37 |
| **Ownley *et al.* 1992** (Figure 2) | **0,018** | **-0,401** | **2,951** | **-6,814** | **6,38** |
| Parkin *et al.* 1985 Figure 1 (limed) | -206,0 | 2515,0 | -6870,0 | 767,0 | 6,40 |
| Fernandez-Calvino et Baath 2010 (Fig. 2/6,9) | -0,040 | 0,597 | -2,606 | 3,591 | 6,67 |
| Fernandez-Calvino et Baath 2010 (Fig. 2/6,5) | -0,050 | 0,773 | -3,615 | 5,316 | 6,76 |

(suite)

| Référence | a | b | c | d | pHo |
|---|---|---|---|---|---|
| Rousk *et al.* 2010 Figure 4 c/n combo | -0,0038 | 0,0647 | -0,3485 | 0,6248 | 6,96 |
| Rousk *et al.* 2010 Figure 4 a/n combo | -0,007 | 0,124 | -0,710 | 1,346 | 7,03 |
| Rousk *et al.* 2010c Figure 1 (alfalfa) | -21,840 | 321,690 | -1246,000 | 1280,000 | 7,17 |
| Rousk *et al.* 2010c Figure 1C | -4,926 | 80,638 | -391,850 | 613,490 | 7,26 |
| Rousk *et al.* 2010c Figure 1 (paille) | -10,099 | 173,390 | -915,780 | 1574,000 | 7,31 |
| Taziebou *et al.* 2004 (Figture 1a) | -15,229 | 251,000 | -1209,000 | 1776,000 | 7,42 |
| Rousk *et al.* 2009 Figure 2A | -1,038 | 17,877 | -93,400 | 155,860 | 7,46 |
| Ownley *et al.* 1992 (Figure 4) | -0,380 | 7,273 | -45,078 | 94,202 | 7,47 |
| Lauber *et al.* 2009 Figure 5 combo | -0,638 | 11,047 | -58,228 | 100,670 | 7,47 |
| Simek et Hopkins 1999 Figure 3 | -4,318 | 72,337 | -357,000 | 546,000 | 7,49 |
| Ownley *et al.* 1992 (Figure 4) | -0,820 | 16,240 | -105,000 | 225,000 | 7,55 |
| Jones *et al.* 2009 (Figure 2-4) | -0,028 | 0,496 | -2,694 | 4,583 | 7,78 |
| Simek *et al.* 2002 Figure 4 | -12,581 | 240,590 | -1451,000 | 2806,000 | 7,85 |
| Simek *et al.* 2002 Figure 7 | -105,000 | 2246,000 | -15702,000 | 36166,000 | 8,13 |
| Simek *et al.* 2002 Figure 5 | -20,414 | 434,370 | -3000,000 | 6792,000 | 8,24 |
| Parkin *et al.* 1985 Figure 1 (unlimed) | 38,0 | -681,0 | 3400,0 | -3589,0 | 8,39 |

Pour que ce type d'analyse aie valeur de diagnostique il faudrait qu'une telle fonction soit plus précise ; le $R^2$ n'est actuellement que de 58% (Figure 1). Il faudrait aussi que cette fonction soit plus applicable aux bactéries en sols arables. Or, à la lumière des données disponibles répertoriées au Tableau 1, cela n'est pas les cas; bon nombre de ces données proviennent de sols forestiers, contaminés, etc. voire très acides recherchés afin d'étudier les acidobactéries.

Cela dit, le pH d'un sol, arable ou non, demeure très structurant pour la microflore bactérienne (Fierer *et al*. 2005 2006 ; Griffiths *et al*. 2011, Nicol *et al*. 2008, Wu *et al*. 2009, Campbell *et al*. 2003). Pourtant, le pHo en ce sens n'est pas aujourd'hui prescriptif en soi. Par exemple, Dick *et al*. 2000 proposèrent un diagnostique acido-basique pour sols arables fondé sur le niveau d'activité des phosphatases acides et alcalines. Or, ce dosage enzymologique, n'est qu'indirectement relié à l'état bactériologique d'un sol ; il est aussi capricieux dans son application *in situ*. Il est aussi difficile de savoir objectivement si le pH d'un sol est (a)normale, ou même (contre)productif. Le pH "normal" du sol serait plutôt callé sur sa capacité de neutralisation acide, valeur sans intérêt pour l'agronome cherchant à ajuster le pH du sol au pHo pour une culture agronomique en particulier (Islam *et al*. 1980). En effet, un pH jugé anormale n'affectera que l'équilibre nutritionnel de la culture ; un tel déséquilibre peut être facilement corrigé la plupart du temps à l'aide de simples apports d'engrais NPK ciblés (Arnon et Johnson 1942).

C'est pourquoi, et malgré l'importance du pH pour les bactéries du sol, la plupart des "kits" d'échantillonnage ne sont que descriptifs et non diagnostiques. Or, et bien que la plupart des inventions concernant des méthodes de diagnostiques, le prélèvement de l'échantillon et/ou la mesure d'une variable, l'activité inventive peut dans les faits aussi se situer au moment de la *constatation* d'un écart significatif lors de cette comparaison, et de l'*attribution* de cet écart à un tableau particulier, *i.e.* ici la phase de décision déductive permettant le diagnostique à finalité curative. Ce type d'invention comporterait donc nécessairement l'élaboration de nouveau standards - de courbes "étalon" par exemples, voire de nouveau modes d'interprétation des écarts par rapport à de tels standards ou références ; de tels courbe normales sont cependant à ce jour difficiles à obtenir, notamment en sols arables.

DIVULGATION DE L'INVENTION

*Problème technique*

Le problème technique découle donc de la nature plus qualitative, subjective en sorte, que quantitative des méthodes d'évaluation de l'état microbiologique et/ou bactériologique de sols arables. Même chiffrés, ces évaluations ne font que décrire en terme de diversité, d'abondance et/ou d'activité ces populations microbiennes sans pour autant diagnostiquer si le fonctionnement micro- et bactériologique du sol est proche ou à son optimum, et/ou particulièrement vulnérable, ou résilient. Ce floue est préjudiciable lors de la préconisation de biofertilisations bactériennes, azotobactérienne au sens de Claude et Fillion (2004) en particulier.

Plus précisément, il est impossible à ce jour de savoir si les populations bactériennes d'un sol en particulier, même bien adaptées au pH du sol établi de longue date ou plus récemment, sont suffisamment *résilientes* pour résister activement à des variations de pH dans le cadre de l'acidité échangeable de ce sol. En effet, un tel manque de résilience implique une sensibilité aiguë des populations bactériennes à des variations de pH par rapport à un pH pourtant supposé optimal.

L'évaluation de cette résilience aura valeur de diagnostique puisque une population bactérienne peu résiliente nécessitera, ou du moins bénéficierait, d'une augmentation, d'un renforcement en sorte, de son contingent, notamment en proximités des résidusphères. Ce type de diagnostique, pourtant fort utile dans le cadre de biofertilisations azotobactériennes par inoculation des résidus de culture cellulosiques au sol (Claude et Fillion 2004), ne fait actuellement pas partie de l'état de la technique accessible à l'homme du métier.

*Solution technique*

La solution technique consiste simplement à combiner le titrage de l'acidité échangeable et la détermination d'un pH optimum (pHo) pour l'activité, la taille et/ou la diversité de la population bactérienne d'un sol. Il est ainsi possible de savoir quel sera l'impact de la variation de pH à entrevoir au cours de la campagne fonction de cette acidité échangeable sur la dynamique desdites populations bactériennes aux alentours de pHo. Dans un premier temps il est défendable de fixer pHo = $pH_{eau}$, les populations bactériennes endogènes étant nécessairement plus ou moins adaptées au pH du sol environnant déterminée à $pH_{eau}$, *i.e.* si la milli - molarité (mM) du KCl est approximativement 0,00. SI les populations bactériennes du sol sont résilientes, leurs dynamiques ne seront pas grandement affectées si le pHo est réduit dans la mesure de l'acidité échangeable du sol. *In contrario*, une baisse appréciable de cette dynamiques à l'intérieur de cette gamme $dpH_{BIS}$ indiquera que lesdites populations sont très peu résilientes ; l'inoculation des résidus de culture pailleux au sol (*eg*. Claude et Fillon 2004) sera donc *préconisable.*

L'évaluation de la résilience des populations bactériennes est donc essentielle à la préconisation de la biofertilisation azotobactérienne. Cette résilience est synonyme d'une résistance de l'activité desdites population à une variation de pH comparable à **celle** de l'acidité échangeable. En effet, cette acidité échangeable bornera les variations de pH les plus susceptibles d'apparaître au cours de l'année, suite par exemple à l'apport de d'engrais, ou encore à des variations dans les niveaux d'activités de la microflore.

Pour évaluer cette résilience il faut simplement imposer ces variations de pH comprises par l'étendue de l'acidité échangeable à une certaine fraction de la microflore bactérienne du sol. Si la perte de ngaBAC est nulle ou faible face à ces variations de pH, lesdites populations seront alors dites résilientes. Concrètement, il s'agit d'une méthode d'évaluation de la résilience de la fraction bactérienne de la microflore d'un sol arable et/ou forestier en termes de diversité, d'activité et/ou d'abondance confrontée à des variations de pH du sol environnant comprenant les étapes suivantes ;

- **échantillonnage** du sol d'une parcelle agronomique et/ou d'îlots agricoles, ou encore d'une parcelle forestière;

- **mesure** de la dynamique de la fraction bactérienne du sol en termes de diversité, d'activité et/ou d'abondance, relative ou absolue, à différentes valeurs de pH comprises à l'intérieure d'une plage de valeurs correspondant à l'étendue de l'acidité échangeable du sol;

- **comparaison** de cette mesure avec des valeurs optimales permettant de constater un écart significatif;

- **diagnostique** de l'état microbiologique et/ou bactériologique du sol par appréciation de cet écart permettant de préconiser une pratique agronomique capable de renforcer ces populations bactériennes

caractérisée en ce que la mesure de la résilience est obtenue par superposition de la courbe décrivant cette variation de la dynamique de ladite fraction bactérienne de la microflore du sol en fonction des susdites différentes valeurs de pH imposées *in vitro* (courbe variable), à une autre courbe fixée horizontalement (courbe fixe) au pH optimal et/ou supposé optimal pour ladite dynamique bactérienne en termes de diversité, d'activité et/ou d'abondance, relative ou absolue. Le nombre de différentes valeurs de pH est lui suffisant pour tracer une courbe décrivant correctement cette relation [ngaBAC : pH], soit ici et par exemple seize (16).

Ledit pHo dans le sol est, i) soit le $pH_{eau}$, soit, ii) le pH maximal effectivement observé à travers une gamme de milieux de culture au pH tamponnés à diverses valeurs permettant ainsi d'observer une augmentation puis une diminution de la dynamique de la fraction bactérienne de la microflore du sol en terme de diversité, d'activité et/ou d'abondance, relative ou absolue. De telles courbes bimodale de la dynamique des bactéries du sol en réponse à une variation graduée du pH ont été rapportées maintes fois dans la littérature scientifique (Tableaux 2 et 3), et leurs réalisations ne pause aucun problème à l'homme du métier.

La plage de valeurs correspondant à l'étendue de l'acidité échangeable du sol est établie par titrage incrémental de l'acidité non résiduelle mais échangeable du sol, et/ou, plus simplement par la différence entre les $pH_{eau}$ et $pH_{KCl}$ ou $pH_{CaCl2}$, ce dernier cas de figure ne donnant comme de raison que deux (2) points pour établir lesdites courbes fixes et variable. Des exemples simulés de tels titrages sont présentés ici au Tableau 3, et graphiquement aux Figures 2, 3 et 4. Il est aussi facile de voir comment des courbes ngaBAC : pH peuvent ainsi être approximées qu'à l'aide de seulement deux (2) points.

L'échantillon de sol peut être avantageusement préalablement pré - incubé en présence d'un minimum de substrat carboné. Ce minimum de substrat carboné est avantageusement calqué sur le Km (coefficient d'affinité) apparent des bactéries du sol pour le glucose, soit ici et par exemple de l'ordre de 70 à 100 uM-glucose, et cela selon la préconisation d'une précédente invention ; EP2314669. Pratiquement, cette pré-incubation est facilité par le recours à un kit d'échantillonnage comportant un filtre macro/microporeux constitué d'un feuilleté multi - plis semi-rigide et/ou souple comprenant quatre couches tel que décrit dans FR 12/01990 (EP 13 366 004.3). Ce faisant, il n'est plus nécessaire de mélanger l'échantillon et le substrat en salle blanche pour éviter que la pré-incubation soit non contaminée.

La mesure de la dynamique, dite ici ngaBAC, de la fraction bactérienne de la microflore du sol ce fait sur la base du métabolisme d'un tel substrat carboné à différentes valeurs de pH comprises à l'intérieure d'un plage de valeurs correspondant à l'étendue de l'acidité échangeable. L'activité catabolique et/ou métabolique est probablement la meilleure mesure de ladite dynamique ngaBAC. De nombreuses appréciations de telles dynamiques ont déjà été rapportées dans littérature (*cf.* Tableaux 1). L'acidité échangeable, elle, est définie ici comme acidité du sol attribuable à l'aluminium et l'hydrogène adsorbés sur le complexe d'échange du sol, et échangeables par d'autres cations tels que le potassium ($K^+$) et/ou le calcium ($Ca^{2+}$). Elle est ici calculée comme la différence entre, i) le pH du sol mesuré dans une solution d'extraction 1:5 (sol / solution), voire 1:10 selon la nature du sol, lorsque la molarité cationique (*eg.* $K^+$ ou $Ca^{2+}$) de cette solution est approximativement de 0,00 mM et ii) lorsqu'elle est de 100, voire 1 000 mM, soit jusqu'à ce que le $pH_{KCl}$ minima se stabilise. L'étendue des valeurs pH propre à cette acidité échangeable est généralement de comprise entre 0,5 et 1,5, voire 2,0 unité pH en dessous du $pH_{eau}$.

La mesure de la dynamique de la fraction bactérienne de la microflore du sol peut aussi ce faire sur la base du dénombrement de cellules bactériennes viables, lui aussi à différentes valeurs de pH comprises à l'intérieure d'un plage de valeurs correspondant à l'étendue de l'acidité échangeable. Ce dénombrement n'est pas nécessairement dépendant d'une quelconque mise en culture in vitro en milieux enrichis, mais peu comprendre l'observation directe, *in situ,* des cellules viable à l'aide d'orange acridine.

Enfin, la mesure de la dynamique de la fraction bactérienne de la microflore du sol peut aussi ce faire sur la base d'un indice de diversité de la microflore bactérienne à différentes valeurs de pH comprises à l'intérieure d'un plage de valeurs correspondant à l'étendue de l'acidité échangeable Paradoxalement, de tels indices, et bien que le pH joue un rôle prédominant dans la biogéographie des bactéries du sol, ne révèlent pas nécessairement des pHo facilement repérables à l'aide de fonctions courbes comme c'est le cas avec des mesures à bases d'activité et/ou de nombre de cellules viables. En dépit des susdites courbes bimodales, les variations de ngaBAC aux alentours de pHo dans le cadre approximatif de dpH sont elles avantageusement décrites simplement par des équations linéaires ; voir en ce sens les exemples d'applications aux Figures 2, 3 et 4.

La comparaison de la mesure de la dynamique bactérienne par superposition des courbes, fixes et variables peut comprendre un intervalle de confiance applicable à ces deux courbes. L'intervalle de confiance pour la courbe variable est fonction de la moyenne à un pH donné, tandis que l'intervalle de confiance pour la courbe fixe est elle invariable et établie à partir du pHo supposé égale ici à $pH_{eau}$ (*i.e.* sol / solution 1:5 à 0,001 mM-KCl). Ce calcul d'intervalles de confiance (ic) est simple et connu de l'homme du métier. Ici, à titre d'exemple, j'ai utilisé l'équation suivante tirée de Wonnacott et Wonnacott (1991, éq. 12-13, p. 438) ;

$$ic = (a + bX_o) +/- t_{0,025} \cdot s \cdot racine(1/n + [(X_o - \dot{X})^2 / \textstyle\sum X^2])$$

lorsque que $(a + bX_o)$ décrit empiriquement la progression d'ngaBAC selon $X_o$ = pH à partir de pHo (voir les deux droites divergentes aux Figures 2, 3 et 4), $t_{0,025}$ est la valeur critique de la statistique t lorsque les ddl ("degrés de liberté") = nombre d'observations (*eg.* ici, n = 16) moins 1, s est l'*écartype* et $(X_o - \dot{X})^2$ la somme des carrés des différences entre $X_o$ et la moyenne X pour les n = 16 observations proposées au Tableau 4.

La comparaison de la mesure de la dynamique bactérienne par superposition des courbes fixes et variables comprend donc un intervalle de confiance applicable à ces deux courbes, au-delà de laquelle la courbe dite variable s'écarte significativement de la courbe fixe.

Il est aussi envisageable de graduer cette résilience en mesurant l'angle, θ, entre les courbes variables et fixes. Or, si la courbe dite variable s'écarte significativement de la courbe fixe, *i.e.* au-delà des bornes établies par les intervalles de confiances en raison d'un angle, θ, important entre ces deux courbes, les populations bactériennes en question sont dites peu ou pas résilientes, et le renforcement de ces populations, par inoculation bactérienne et/ou azotobactérienne par exemple, est *préconisable.*

Le diagnostique de l'état microbiologique et/ou bactériologique sur la base de l'angle θ, mesure de la résilience des populations bactériennes de l'échantillon du sol, peut aussi être pondéré à l'aide d'une mesure du phosphore inorganique, Pi, en solution d'extraction avant et après le titrage de l'acidité échangeable du sol. Pour ce faire, on utilise une relation entre la teneur en Pi en **solution** d'extraction de l'échantillon avant et après titrage de l'acidité échangeable, avantageusement linéaire et décrite par une simple régression linéaire dont la pente est comprise entre des valeurs de 0,50 à 0,25, plus particulièrement de 0,40 à 0,30, et avantageusement de 0,38 +/- 0,05. Cette pondération du diagnostique de l'état microbiologique et/ou bactériologique sur la base de l'angle θ, mesure de la résilience des populations bactériennes de l'échantillon du sol est ensuite effectué comme suit;

> si $Pi_{CaCl2, KCl}$ observée est supérieur à celle prédite à partir de relations [$Pi_{Ca}$:$Pi_{eau}$] telles que proposées au Tableau 5, et sachant que $PiCa < Pieau$, le pH effectif est probablement plus grand que le $pH_{CaCl2}$, ou $pH_{KCl}$, ce vecteur va atténuer la pleine réalisation de l'acidité échangeable,

> si $Pi_{CaCl2, KCl}$ observée est égale à celle prédite, aucune information nouvelle est apportée,

> si $Pi_{CaCl2, KCl}$ observée est inférieure à celle prédite, et sachant encore une fois que $Pi_{CaCl2, KCl} < Pi_{eau}$, le pH effectif, en puissance en sorte, est vraisemblablement plus faible que le $pH_{CaCl2}$, ou $pH_{KCl,}$ observé, et ce vecteur va accentuer la pleine réalisation de l'acidité échangeable,

de manière à ce que en combinaison avec les mesures de θ, il est donc possible d'établir un diagnostique de l'état bactériologique du sol pour les situations suivante;

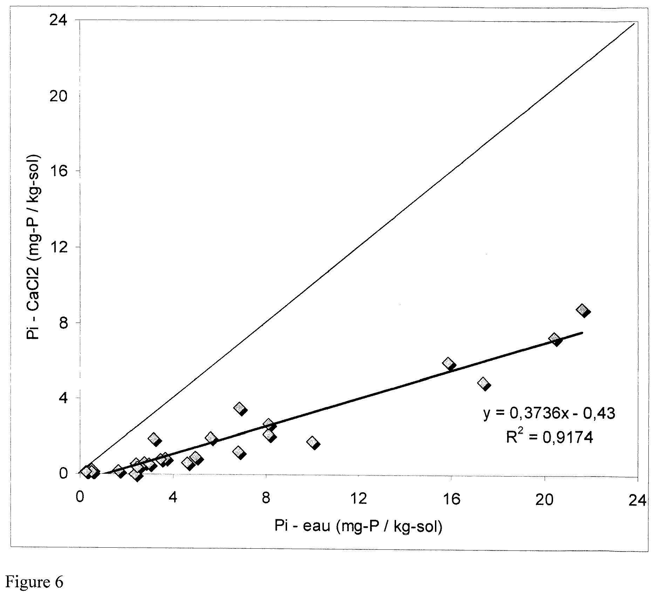

> lorsque θ est faible, et si $Pi_{CaCl2, KCl}$ est moins importante que prédite, la bonne résilience des bactéries du sol sera assistée d'un pH en puissance dont le vecteur positif tend à réduire le risque de décrochage de ngaBAC. Si $Pi_{CaCl2, KCl}$ est plus importante que prédite selon l'une ou l'autre des équations au Tableau 5 (eg. Figure 6), la bonne résilience des bactéries du sol devra affronter un pH en puissance à la baisse (vecteur négatif) amplifiant la baisse des pH déjà probable dans le cadre de l'acidité échangeable mesuré.

> lorsque_est importante, et si $Pi_{CaCl2, KCl}$ est moins importante que prédite, malgré une piètre résilience des bactéries du sol, la probabilité qu'elles aient à affronter la plein réalisation de l'acidité échangeable est réduite proportionnellement à la différence entre $Pi_{CaCl2, KCl}$ observée et prédite selon l'une ou l'autre des équations au Tableau 5 (eg. Figure 6). Si $Pi_{CaCl2, KCl}$ est plus importante que prédite, la piètre résilience des bactéries du sol est d'autant plus conséquente que le pH en puissance est doté d'un vecteur négatif augmentant la probabilité d'une plein réalisation de l'acidité échangeable du sol.

Avantages apportés

L'invention permet de préconiser l'inoculation des résidus de culture pailleux au sol en fonction d'une mesure objective de la résilience des bactéries du sols confrontées à des variations de pH à travers la campagne sommes toutes normales puisque dérivées de la courbe de titrage de l'acidité échangeable du sol. Ce type de préconisation n'est pas possible à l'aide des autres kits de diagnostiques de l'état microbiologique de sols arables répertoriés dans la base de connaissance.

Sigles et définitions

Acidité échangeable (du sol), alias ici dpH ; acidité du sol attribuable à l_aluminium et l_hydrogène adsorbés sur le complexe d'échange du sol, et échangeables par d_autres cations tels que le potassium ($K^+$) et/ou le calcium ($Ca^{2+}$).

Dynamique des populations bactériennes du sol (ngaBAC) ; changements constatés à travers le temps et/ou en fonction d'un variations de paramètres physico-chimiques, tel que le pH notamment, du nombre de cellules bactériennes (n), leurs masses et/ou leurs abondances relative à celles des autres fractions de la microflore du sol (g), leurs diversité génomique et/ou phénotypiques et, surtout, leurs activités métaboliques et/ou cataboliques (a).

Fraction bactérienne (de la microflore du sol (BAC); biomasse bactérienne séparée physiquement, par centrifugation d'une suspension aqueuse du sol (cf. Bakken 1985), ou chimiquement, sans nécessairement impliquer la mise en culture des cellules bactériennes qui pourrait biaiser malencontreusement sa composition au profit des bactéries les plus copiotrophes.

Résilience (des populations bactériennes du sol) ; caractéristique biologique, notamment ici des bactéries sol, capables de maintenir une certaine dynamique optimale en dépit de variations de pH comprises par l'acidité échangeable, et plus particulièrement ici en présence de pH infra optimaux. Ici, cette résilience est mesurée par superposition d'une courbe décrivant cette variation de ngaBAC en fonction des valeurs de pH imposées in vitro (courbe variable), à une autre courbe fixée horizontalement (courbe fixe) à partir d'un pH optimal (pHo) en terme de ngaBAC ; il est aussi possible de graduer cette résilience en mesurant l'angle, _, entre les courbes variables et fixes

pHo : mesure de l'acidité active en terme de pH à laquelle la dynamique des populations bactériennes du sol (ngaBAC) est la plus grande, voire optimale ; mathématiquement, cela équivaut à une première dérivée de l'équation de la courbe décrivant la relation [ngaBAC : pH] = 0,00.

$pH_{eau}$, $pH_{KCl}$, $pH_{CaCl2}$ : Mesure de l'acidité du sol en terme de pH à l'aide d'une solution d'extraction strictement aqueuse (eau), ou saline comportant différentes concentrations de KCl ou de $CaCl_2$ selon le cas; les concentrations de ces solutions salines et les ratios sol/solution peuvent varier, et ne font pas unanimité (Tableau 3). Les $pH_{KCl}$ et $pH_{CaCl2}$ peuvent aussi être obtenus par titrage acido-basique.

**$Pi_{eau}$, $Pi_{KCl}$, $Pi_{CaCl2}$** : Mesure (mg-Pi / kg-sol) des teneurs en phosphore inorganique (Pi) exactement dans les mêmes conditions, ou plus exactement solutions d'extraction, que les susdits **$pH_{eau}$, $pH_{KCl}$, $pH_{CaCl2}$**.

**Solution d'extraction** : Mélange sol/solution dont la molarité en KCl ou $CaCl_2$ est plus ou moins importante de manière à affecter les concentrations de protons $H^+$ (pH) ou d'anions phosphate $PO_4^-$ (Pi). La composition de ces solutions d'extraction, tant en terme des ratios sol/solution qu'en terme desdites molarités, ne fait pas unanimité ; voir en ce sens *infra* le Tableau 3.

## BRÈVES DESCRIPTION DES FIGURES

**Figure 1** : Courbure, *i.e.* la deuxième dérivée (f''(x)) de fonctions empiriques ngaBAC = f(pH) lorsque pH = pHo. L'ensemble des valeurs empiriques (Tableau 1) peuvent ainsi être rapportées sur une même figure. Les populations bactériennes les plus stables *in situ* affichent ainsi des courbures les moins prononcées (négatives), soit aux alentours d'un pHo in vitro de 6,00 ; de par et d'autre de cet optimum, les courbures de plus en plus négatives dénotent des populations bactériennes de plus en plus sensibles à des variations du pH du sol. Il s'agit d'une fonction ngaBAC = f(pH) polynomiales de deuxième degré dont le $R^2$ est de 58%, et comporte ici ***un** intervalle de précision* pour un pH +/- 20%, soit un dpH d'environ 1,00 +/- 0,44 unité de $pH_{sol}$

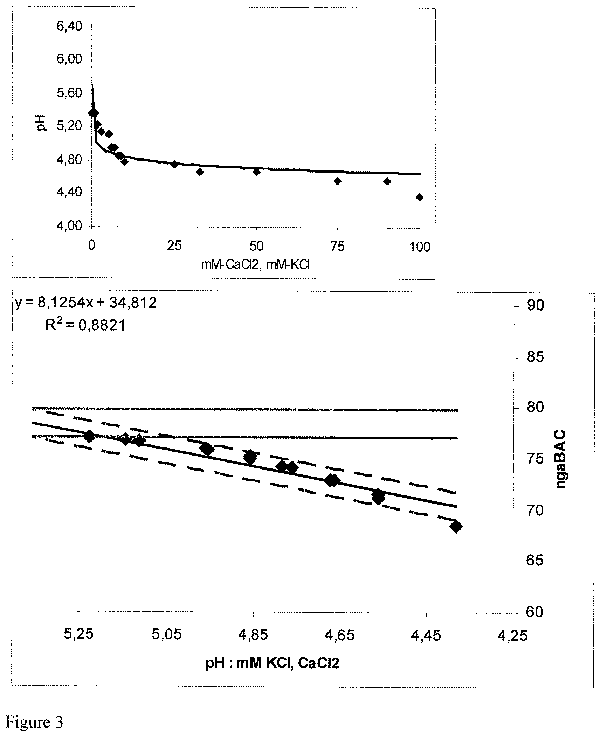

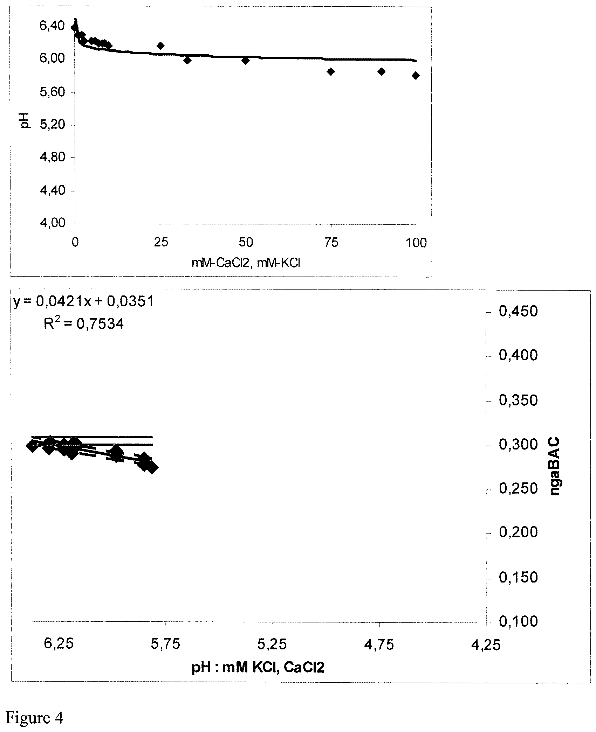

**Figure 2** : Activité catabolique des populations bactériennes du sol à l'égard du glucose en réponse à une diminution du pH (Tableau 4). La plus petite figure (haut) représente la courbe de titrage de l'acidité échangeable, généralement une puissance négative de la mM-KCl (ou $CaCl_2$) de la solution d'extraction. La figure du bas représente elle le niveau d'activité catabolique, ici à l'égards du glucose, de la fraction bactérienne du sol lorsque confrontée à un tel dpH. L'exemple proposée est basée sur la dynamique ngaBAC : pH selon les données publiées par Baath 1998 (*cf.* tableau 1 *op. cit.* ; taux d'incorporation de la leucine). A noter que l'abscisse est inversée et débute précisément à pHo, *i.e.* le pH optimum supposé pour l'activité de ces populations bactériennes, soit ici 5,85 *(cf.* ligne pointillée horizontal). Ici, comme aux Figures 3 et 4, ces deux courbes, variable et fixe (horizontale), forment une angle, $\theta$, et sont bordées d'intervalles de confiance (ic, 95%).

**Figure 3** : *idem* à Figure 2, mais ici l'exemple proposée ici est basée sur la dynamique ngaBAC : pH selon les données publiées par Baath *et al*. 1995 (*cf.* figure 6 *op. cit.* ; taux d'incorporation de la thymine). A noter encore une fois que l'abscisse est inversée et débute précisément à pHo, *i.e.* le pH optimum supposé pour l'activité de ces populations bactériennes, soit ici 5,36 (*cf.* ligne pointillée horizontal).

**Figure 4** : *idem* aux Figures 2 et 3, mais ici l'exemple proposée ici est basée sur la dynamique ngaBAC : pH selon les données publiées par Ownley *et al*. 1992 (*cf.* figure 2 *op. cit.* ; taux d'inhibition de l'activité fongistatique de *Pseudomonas* spp.). A noter que l'abscisse est inversée et débute précisément à pHo, *i.e.* le pH optimum supposé pour l'activité de ces populations bactériennes, soit ici 5,65 (*cf.* ligne pointillée horizontal).

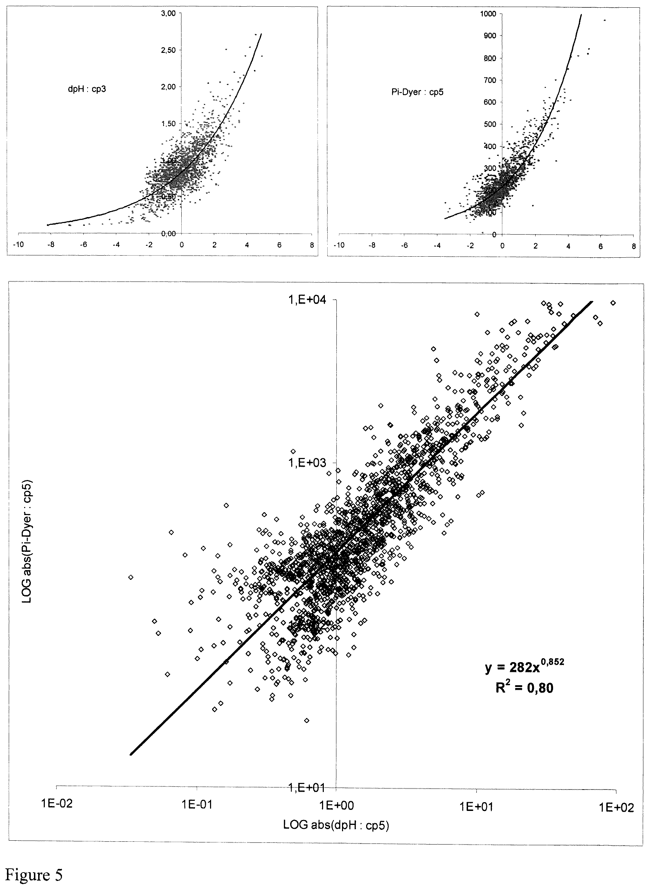

**Figure 5** : A priori, les valeurs pédologiques d'acidité échangeable et de phosphore inorganique, ici $Pi_{Dyer}$, bien peu ou pas corrélées en l'état, peuvent être mises en relations via une composante principale (cp) issues **_d'_**analyses par composante principale (ACP), ici **_d'un_** ensemble de 1699 moyennes cantonales des principales variables pédologiques renseignées dans la base de données des analyses de terres (BDAT ; Gissol 2006). Ici, la cp3 est la mieux corrélées avec l'acidité

échangeable, et la cp5 avec PiDyer ; une fois rapporté à cp5, l'acidité échangeable peut ainsi être mise en relation avec PIDyer / cp5.

**Figure 6** : Représentation graphique de données empiriques publiées, ici celles de Kleinman et al. 2001 (op. cit., tableau 6) décrivant la relation linéaire entre $Pi_{CaCl2}$. et $Pi_{eau}$. Bien que les intersections à $X_0$ (coefficients a) sont assez variables du fait d'unités différentes (cf. Tableau 5), les pentes de ce type de droites sont assez constantes, soit au alentours de 0,38 +/- 0,05. Connaissant $pH_{CaCl2, KCl}$ et $pH_{eau}$ il est ainsi possible de croiser ces deux couplets, et pondérer notre mesure de l'acidité échangeable, dpH (voir texte).

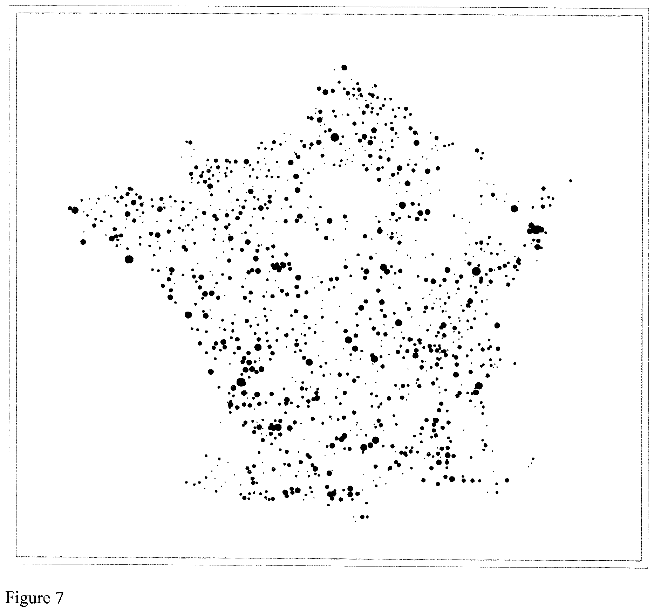

**Figure 7** : Représentation géostatique des acidités échangeables, dpH, supérieures à la **moyenne** nationale française de 0,57 unité [$pH_{eau}$ - $pH_{KCl}$]. Chaque "bulle" représente une moyenne cantonales issues de la Base des données d'analyses de terre (BDAT ; Gissol 2006). Seules les bulles manifestement sphériques, i.e. hors les points visiblement sans dimensions, représentent des dpH $\geq$ 0,57. Le risque que la résilience des populations bactériennes des sols soient ainsi mises à l'épreuve est a priori assez répandue, et qu'il y parfois attroupement des dpH les plus élevés, i.e. ici des bulles les plus grandes.

## MODE DE RÉALISATION DE L'INVENTION

Détermination de l'acidité échangeable du sol

La variation de pH caractéristique de la l'acidité échangeable est déterminée par simple titrage. Une fois ce titrage complété, les pH mesurés dans les solutions d'extraction seront inférieurs de 0,5 à 1,5 unités au $pH_{eau}$ puisque les ions $K^+$ s'échangent avec des ions $H_3O^+$ qui n'étaient pas dissociées en solutions aqueuse. Avantageusement, la solution d'extraction est progressivement titrée par l'apport dosé d'une solution concentrée de KCl; le rapport massique initiale de cette solution d'extraction peut être ajusté, à 1/10 par exemple, en fonction de la nature du sol de manière à permettre une agitation continuelle lors du titrage. Après chaque apport incrémentiel de KCl, la solution d'extraction est incubée sous agitation assez longtemps et jusqu'à la nouvelle mesure pH (à la baisse) soit stabilisée.

Si le sol ne se prête pas à un tel titrage sous agitation continuelle, il est aussi possible de préparer individuellement un série de solutions d'extraction à base de KCl ou de $CaCl_2$ de 0,01 à 1000 mM selon le cas (cf. Tableau 3), applicables à un rapport massique sol/solution 1/5 (norme ISO 10390), ou encore 1/10. On peut ainsi disposer un aliquote de chacune de ces suspensions dans des micropuits afin d'en mesurer individuellement le pH, simultanément, après une période d'incubation permettant la stabilisation de ces mesures. Ad minima, l'acidité échangeable peut ici être définie comme la différences entre les $pH_{eau}$ et $pH_{KCl}$ au sens entendu.

Dans la pratiques, il n'y a pas unanimité, et plusieurs combinaisons solutions d'extraction : molarité KCl, $CaCl_2$ possibles sont rapportées dans la littérature (Tableau 3). D'une manière ou d'une autre, le but et la détermination de l'acidité échangeable, et donc accessoirement le Pi soluble dans l'eau une fois cette acidité échangeable neutralisé.

**Tableau 3** : Solutions d'extraction et ratio sol/solution pour la détermination de l'acidité active ($pH_{eau}$) et/ou échangeable ($pH_{KCl}$, $pH_{CaCl2}$), et donc les concentrations de phosphore inorganique (Pi) en de telles situations.

| | | molarité de la solution | |
|---|---|---|---|
| mesure d'acidité du sol | ratio sol / solution | KCl | CaCl2 |
| pHeau | 1/1 | na | na |
| pHeau | 1/5 | na | na |
| pHKCl | 1/1 | 0,10 | na |
| pHKCl | 1/5 | 1,0 | na |
| pHCaCl2 | 1/10 | na | 0,01 |

Détermination de la dynamique, ngaBAC, des bactéries du sol

Il n'est pas nécessaire de mettre en culture la flore bactérienne du sol. Il s'agit simplement de la séparer du sol par centrifugation selon un mode opératoire établi, par exemple, par Bakken et al. 1985. Des méthodes à base de fongicides non bactéricides sont aussi envisageable, l'échantillon de terre après traitement avec de tels fongicides ne comportant essentiellement qu'une fraction bactérienne de la microflore du sol.

Une série d'aliquotes de ce cette fraction **bactérienne** est déposée dans autant de micropuits, avantageusement disposés en galeries, un peu à la mode des galeries API™ et/ou Biolog™. Ces aliquotes sont enrichis d'un substrat carboné, aussi simple que le glucose s'il le faut, mais aussi avantageusement de substrats plus complexes, voire des acides **aminés**. Le pH de chaque aliquote peut ainsi être ajustée à l'aide d'une solution tampon de manière à reproduire la gamme de pH observée lors du titrage de l'acidité échangeable ; la dynamique d'utilisation desdits substrats carbonés peut dont être observée après une certaine période d'incubation, généralement de l'ordre de 24 à 48 heures. Le pH du puit affichant la plus grande utilisation desdits substrats indiquera le pH optimum (pHo) au sens entendu. De nombreux exemples de telles déterminations de pHo pour les microflores bactériennes existent (Tableau 1) ; elles impliquent de nombreux types de substrats C, parfois même marqués d'isotopes.

La dynamique des populations bactériennes peut être exprimées en termes d'abondance, d'abondance relative, de biomasse et/ou nombre de cellules bactériennes, ou encore d'activité métabolique. Pour faire simple, et par rapport à une valeur initiale à pHo, nous parlerons ici de ngaBAC, i.e. du nombre de cellules (n), leurs biomasses (g), et/ou leurs activités métaboliques, ou plus exactement cataboliques (a) s'agissant surtout de la disparition par consommation d'un quelconque substrat carbonés. Ces mesures sont donc avantageusement exprimées par rapport aux valeurs ngaBAC observées à pHo.

Comparaison des dynamiques ngaBAC

Pour évaluer la résilience des bactéries du sol à une variation de pH comprise par l'étendue de l'acidité échangeable du sol il s'agit simplement de reporter en abscisse cette gamme de pH, et en ordonnée les différentes valeurs ngaBAC observées in vitro dans chacun des micropuits à de tels pH. Aux Figure 2, 3 et 4 je propose une série de telles comparaisons à partir des données de Baath 1998, Baath et al. 1995 et Ownley et al. 1992 (Tableau 4).

**Tableau 4** : Titrage de l'acidité échangeable d'un sol d'où proviendrait trois populations bactériennes ici à titre d'exemples d'application de l'invention au diagnostique bactériologiques des sols arables.

selon les données de Bääth 1998 (cf. Figure 2)

| mM-KCl | pH : mM-KCL → pH in vitro | npaBAC | + ic 95% | - ic 95% |
|---|---|---|---|---|
| 0,001 (pHo) | 5,85 | 0,213 | 0,244 | 0,210 |
| 2 | 5,63 | 0,210 | 0,227 | 0,193 |
| 3 | 5,63 | 0,208 | 0,227 | 0,193 |
| 5 | 5,63 | 0,205 | 0,227 | 0,193 |
| 6 | 5,57 | 0,208 | 0,222 | 0,189 |
| 7 | 5,57 | 0,203 | 0,222 | 0,189 |
| 8 | 5,57 | 0,208 | 0,222 | 0,189 |
| 9 | 5,39 | 0,197 | 0,209 | 0,175 |
| 10 | 5,21 | 0,192 | 0,195 | 0,162 |
| 25 | 5,21 | 0,189 | 0,195 | 0,161 |
| 33 | 5,01 | 0,172 | 0,180 | 0,146 |
| 50 | 4,77 | 0,142 | 0,162 | 0,129 |
| 67 | 4,77 | 0,143 | 0,162 | 0,128 |
| 75 | 4,76 | 0,143 | 0,162 | 0,128 |
| 90 | 4,74 | 0,142 | 0,160 | 0,126 |
| 100 | 4,65 | 0,123 | 0,153 | 0,120 |

selon les données de Bääth et al. 1995 (cf. Figure 3)

| mM-KCl | pH : mM-KCL → pH in vitro | ngaBACe | + ic 95% | - ic 95% |
|---|---|---|---|---|
| 0,001 (pHo) | 5,36 | 77,219 | 80,04 | 76,89 |
| 2 | 5,30 | 77,462 | 79,46 | 76,32 |
| 3 | 5,27 | 77,040 | 79,23 | 76,09 |
| 5 | 5,06 | 76,488 | 77,37 | 74,24 |
| 6 | 4,97 | 75,927 | 76,57 | 73,44 |
| 7 | 4,76 | 74,147 | 74,69 | 71,56 |

(suite)

selon les données de Bääth et al. 1995 (cf. Figure 3)

| mM-KCl | pH : mM-KCL → pH in vitro | ngaBACe | + ic 95% | - ic 95% |
|---|---|---|---|---|
| 8 | 4,75 | 73,800 | 74,59 | 71,47 |
| 9 | 4,73 | 73,509 | 74,43 | 71,31 |
| 10 | 4,70 | 73,356 | 74,19 | 71,06 |
| 25 | 4,56 | 71,428 | 72,91 | 69,78 |
| 33 | 4,56 | 71,361 | 72,91 | 69,78 |
| 50 | 4,56 | 71,449 | 72,91 | 69,78 |
| 67 | 4,56 | 71,222 | 72,91 | 69,78 |
| 75 | 4,56 | 71,364 | 72,91 | 69,78 |
| 90 | 4,40 | 68,989 | 71,55 | 68,42 |
| 100 | 4,35 | 67,979 | 71,13 | 67,99 |

selon les données de Ownley et al. 1992 (cf. Figure 4)

| mM-KCl | pH : mM-KCL → pH in vitro | ngaBAC | + ic 95% | - ic 95% |
|---|---|---|---|---|
| 0,001 (pHo) | 6,38 | 0,299 | 0,318 | 0,299 |
| 2 | 6,38 | 0,297 | 0,318 | 0,299 |
| 3 | 6,09 | 0,298 | 0,300 | 0,281 |
| 5 | 6,09 | 0,297 | 0,300 | 0,281 |
| 6 | 6,09 | 0,299 | 0,300 | 0,281 |
| 7 | 6,08 | 0,295 | 0,300 | 0,281 |
| 8 | 6,08 | 0,291 | 0,300 | 0,281 |
| 9 | 5,82 | 0,276 | 0,283 | 0,264 |
| 10 | 5,78 | 0,275 | 0,281 | 0,262 |
| 25 | 5,78 | 0,279 | 0,281 | 0,262 |
| 33 | 5,76 | 0,276 | 0,279 | 0,260 |
| 50 | 5,69 | 0,259 | 0,275 | 0,256 |
| 67 | 5,69 | 0,263 | 0,275 | 0,256 |
| 75 | 5,62 | 0,251 | 0,270 | 0,251 |
| 90 | 5,61 | 0,258 | 0,270 | 0,251 |
| 100 | 5,61 | 0,254 | 0,270 | 0,251 |

Les dynamiques dites ngaBAC fonction de pH du sol commentées au Tableau 1 sont elles décrites par des équations polynomiales empiriques dont les coefficients sont *rapportés* au Tableau 2. Trois (3) cas de figures ont été simulés afin de démontrer l'applicabilité de l'invention au diagnostique des sols; ces dynamiques [ngaBAC : pH] sont chiffrées au Tableau 4 et décrites graphiquement aux Figures 2, 3 et 4. La plage de valeur pH ainsi généré est par la suite imposée à la fraction bactérienne de la microflore extraite de l'échantillon de terre. Il est aussi possible de pré - incuber ce type d'échantillon en présence d'un minimum de glucose (*eg.* 70 à 100 uM; EP 10 366 005.6). Étant donné que le $pH_{eau}$ est généralement le pHo (Fernandez-Calvino et Bääth 2010) pour le fonctionnement de ces populations bactériennes, la dynamique ngaBAC va nécessairement décroître plus ou moins rapidement en fonction de la diminution du pH selon la mM du KCl imposé (Figures 2, 3 et 4) ; moins cette décroissance est rapide à partir de pH initial (pHo), plus les populations bactériennes sont résilientes. Des intervalles de confiances (95%) applicables aux régressions [ngaBAC : pH] ont été calculées et représentées graphiquement aux Figures 2, 3 et 4.

Concrètement, ce type de dynamique pourra être reproduit plus simplement en suivant la consommation à un moment donné de substrats carbonés aussi simples que le glucose, voire le xylan et/ou le mannitol, substrats apparaissant en sol du sol lors de la dégradation in situ des résidus de culture pailleux, voire stratégique pour la dynamique des populations azotobactériennes (*cf.* Campbell *et al.* 2003, Pankratov *et al.* 2008).

## APPLICATION AGRONOMIQUE DE L'INVENTION

L'invention est applicable à la biofertilisation, notamment via l'azotobactérisation des résidus de culture pailleux au sol (Claude et Fillion 2004), voire la bactérisation d'engrais organominéraux (Claude et Giroux 2006).

L'échantillon de sol est prélevé sur la parcelle à l'aide d'un kit d'échantillonnage, avantageusement capable de préserver l'intégrité microbiologique de l'échantillon jusqu'à l'arrivé au laboratoire (eg. FR12/01990, EP13/366004.3). Il est ainsi possible d'orienter l'analyse en pré - incubant l'échantillon de terre avec une solution de glucose d'au plus 100 uM (EP 2314669). Cela aura pour effet de favoriser le développement des bactéries les plus réactives à d'aussi faibles teneurs en glucose, ces bactéries étant en principe les plus conséquentes pour la valorisation azotobactérienne des résidus de culture pailleux au sol (EP 2314669), notamment en présence de faibles doses de sucres simples surajoutées au moment de l'inoculation (EP 2213154)

Il ne s'agit pas tellement de comparer des valeurs observées avec des valeurs de références, mais de prévoir l'effet de la réalisation de l'acidité échangeable sur la dynamique des populations bactériennes du sols, dite ici ngaBAC. Il faut donc comparer la courbe décrivant la dégradation (consommation) du substrat à travers une gamme de pH imposés correspondant à l'étendue de cet acidité échangeable, à une courbe horizontale fixée initialement à pHo. Chacune de ces courbes sont bordées d'intervalles de confiance (95%).

A noter que pHo est ici a *priori* défini comme $pH_{eau}$. En effet, selon les données de Frenandez-Calvino et Bääth (2010), le $pH_{eau}$ est généralement optimal pour la dynamique des populations bactériennes du sol. De plus, la résilience est ici graduée à partir de $pH_{eau}$ et à l'encontre de pH plus acides, et non basiques. En effet, en bactériologie du sol, tout semble ce jouer à gauche, i.e. lorsqu'en présence de pH plutôt acides *(cf.* figure 2, Fernandez-Calvino et Bääth 2010) ; c'est l'acidification du sol, ou plus particulièrement ici la réalisation de l'acidité échangeable, qui est la plus souvent conséquente à court terme *in situ* au sein des *microhabitats* du sol. Les diminutions d'activités métaboliques sont aussi plus marquées, plus abruptes, suite à une diminution, qu'à une augmentation, du pH du sol *(cf.* figure 2, *op. cit*.). Évaluer la résilience d'une quelconque fraction de la flore bactérienne du sol équivaut donc à lui imposer des pH de plus de plus faibles par rapport à son supposé pHo (pHeau).

Donc, si la courbe [ngaBAC : pH] s'éloigne appréciablement de cet intervalle de confiance fixé sur pHo, *i.e.* au-delà des intervalles de confiances au pH les plus faibles, les populations bactériennes sont considérées comme peu résilientes. Des intervalles de confiances sont proposés de par et d'autre de la courbe (moyenne) afin de permettre d'inférer si la dynamique de dégradation de substrats résiste à la baisse de pH imposée. Dans ce cas-ci, le pHo est indiqué par une ligne horizontale (Figures 2, 3 et 4). Ces populations bactériennes peu ou pas résilientes pourraient donc être renforcées par l'application d'un inoculum bactérien, avantageusement *Azotobacteraceae,* contribuant à maintenir leurs dynamisme advenant des pH faibles. A noter que ces populations réintroduites sont avantageusement les plus réactives à de faibles concentrations d'oses et/ou d'osides tels que le glucose *(cf.* EP2314669, EP2213154) et pourront d'autant plus s'implanter rapidement et mettre en échec cet acidité échangeable.

A titre exemplaire, voir ici les 3 cas de figures aux Figures 2, 3 et 4 (Tableau 4). A noter que la résilience de ces populations bactériennes idéales ne sont pas très bonnes, leurs dynamiques ngaBAC ne résistant pas pleinement à une certaine réduction de pH simulée, baisse de pH pourtant de l'ordre de grandeur des acidités échangeables généralement mesurées dans ce types de sols, soit ici de 0,8 à 1,2 unité pH en dessus de pHo (pHeau). La préconisation d'une inoculation Solativ™ serait donc ici de mise dans tous les cas.

Pondération des mesures de résilience in situ des BAC

La plausibilité de l'invention est améliorée si on intègre les teneurs du sol en phosphore inorganique, Pi (mg-P/kg-sol), et cela à différentes teneurs en KCl et/ou $CaCl_2$ de la solution d'extraction. En effet, et bien qu'il existe foncièrement une corrélation entre Pi et dpH, celle-ci est très confondue (masquée) par les multiples interactions entre les variables pédologiques, d'où la nécessité de recourir à des analyses par *composantes principales* (cp) pour mettre en évidence ces corrélations [Pi : dpH] intrinsèques (Figure 5) mais noyées en sorte au sein de l'écosystème pédologique. Il est donc peu recommandable d'inférer simplement quel devrait être, en moyenne, Pi pour un certain dpH, ou vice et versa, dpH étant donné un certain Pi, directement à partir d'une corrélation telle que celles à la Figure 5. Il faut plutôt chercher à *pondérer* dpH selon Pi, cette pondération pouvant ainsi soit accentuer, soit atténuer le diagnostique. Je m'explique.

Selon Houba et al. 1986, et nombre d'autres auteurs, il est possible d'extraire le Pi du sol simplement avec une solution de 0,01M de $CaCl_2$. Or, ces extractions de $Pi_{CaCl2}$ sont corrélées avec celles du Pi qu'avec de l'eau ($Pi_{eau}$); quelques une de ces corrélations glanées dans la littérature scientifique ont été compilées (Tableau 5), et l'une d'entre elles (Kleinman et al. 2001) présentée à la Figure 6. Ce qu'il y a d'étonnant c'est la constance des pentes de ces corrélations, soit en moyenne et aux alentours de 0,38 +/- 0,05. Il est donc possible de prédire ce que sera $Pi_{CaCl2}$, et/ou vraisemblablement $Pi_{KCl}$, en fonction d'une première mesure de $Pi_{eau}$ au début du susdit titrage de l'acidité échangeable, ou plus simplement lors de la détermination du $pH_{eau}$, afin d'obtenir dpH. La concentration en KCl ou $CaCl_2$ de la solution d'extraction va donc agir tant sur le pH mesuré que sur la teneur Pi de cette solution ; les deux mesures peuvent donc être a priori rapprochées. Étant donné ladite constance dans la relation [$Pi_{CaCl2}$:$Pi_{eau}$], la teneur du Pi dans un extrait de la solution du sol à 1M KCl (1/5) ou de 100 m$MCaCl_2$ (1/10) sera approximativement 1/3 celle dans une solution aqueuse à 0M KCl ou $CaCl_2$ (Figure 6).

**Tableau 5** : Compilation de coefficients des pentes, b, de régressions linéaires décrivant la relation entre $Pi_{CaCl2}$ et $Pi_{eau}$. Sont spécifiés les tableaux ou figures d'où proviennent les données dans chacune des références, ainsi que les unités, R2 et le nombre d'observations (n). Le coefficients a n'est pas de grande utilité ici puisque les unités diffèrent.

| Référence | source op. *cit.* | unités | $Pi_{CaCl2} = a + b \cdot Pi_{eau}$ *a* | b | R2 | n |
|---|---|---|---|---|---|---|
| Kleinman et al. 2001 | tableau 6 | mg-P / kg-sol | *-0,43* | 0,3736 | 0,92 | 23 |
| Devau et al. 2009 | figure 1 | ug-P / kg-sol | *31* | 0,3534 | 1,00 | 18 |
| Pautler et Sims 2000 | figure 3a | mg-P / kg-sol | *-1,63* | 0,3499 | 0,93 | 17 |
| Pautler et Sims 2000 | figure 5b | mg-P / kg-sol | -3,261 | 0,4839 | 1,00 | 17 |
| Koopsmans et al. 2004 | tableau 3 | ug-P / L solution de sol | *0,0945* | 0,3402 | 0,89 | 9 |
| Koopsmans et al. 2004 | tableau 3 | ug-P / L solution de sol | *-0,0829* | 0,3973 | 0,95 | 9 |
| | | | **b moyen** → | 0,38 | | |
| | | | **écartype b** → | **+/- 0,05** | | |

Considérons maintenant ce dpH comme une vecteur qui pourra soit s'additionner ou ce soustraire à un autre vecteur représentant la tendance qu'à le pH d'un sol à atteindre une certaine valeur. Si celle-ci est proche ou égale à une mesure prédite, il n'y a pas de nouvelle information apportée. Si elle est plus faible que prédite, le vecteur négatif vers un pH plus acide s'additionnera à celui de dpH et le risque que l'angle $\theta$ entre les courbes fixe et variable (*eg.* Figures 2, 3 et 4) soit conséquente en terme de ngaBAC est ainsi accru. Dans le cas contraire, le vecteur positif vers un pH plus alcalin se soustrait au, annule en sorte, le vecteur négatif dpH, et le risque que l'angle $\theta$ soit ainsi conséquente est diminué. Dans les faits il y a trois cas de figures ;

➢ le $Pi_{CaCl2, KCl}$ observé est supérieur à celui prédit à partir d'une relation [$Pi_{Ca}$:$Pi_{eau}$], eg. Figure 6 (cf. Tableau 5). Dans ce cas, sachant que $Pi_{CaCl2}$ ou $Pi_{KCl} < Pi_{eau}$, le pH effectif est probablement plus grand que le $pH_{CaCl2}$, ou $pH_{KCl}$, et ce vecteur va atténuer la pleine réalisation de l'acidité échangeable.

➢ le $Pi_{CaCl2, KCl}$ observé est égale à celui prédit et aucune information nouvelle est apportée.

➢ le $Pi_{CaCl2, KCl}$ observé est inférieur à celui prédit. Dans ce cas, et sachant encore une fois que $Pi_{CaCl2, KCl} < Pi_{eau}$, le pH effectif est vraisemblablement plus faible que le $pH_{CaCl2}$, ou $pH_{KCl}$, observé, et ce vecteur va accentuer la pleine réalisation de l'acidité échangeable.

En combinaison avec les diverses mesures de $\theta$, il est donc possible d'apercevoir un abaque applicable au diagnostique de l'état bactériologique du sol;

➢ les dBAC:dpH ($\theta$) sont faibles : Si $Pi_{CaCl2, KCl}$ est moins importante que prédite, la bonne résilience des bactéries du sol sera assistée d'un pH en puissance dont le vecteur positif tend à réduire le risque de décrochage de ngaBAC. Si $Pi_{CaCl2, KCl}$ est plus importante que prédite par l'une ou l'autre des équations au Tableau 5 (eg. Figure 6), la bonne résilience des bactéries du sol devra affronter un pH en puissance à la baisse (vecteur négatif) amplifiant la basse des pH déjà probable dans le cadre de l'acidité échangeable mesuré.

➢ les dBAC:dpH ($\theta$) sont importants : Si $Pi_{CaCl2, KCl}$ est moins importante que prédite, malgré une piètre résilience des bactériens du sol, la probabilité qu'elles aient à affronter la plein réalisation de l'acidité échangeable est réduite proportionnellement à la différence entre $Pi_{CaCl2, KCl}$ observée et prédite selon l'une ou l'autre des équations au Tableau 5 (eg. Figure 6). Si $Pi_{CaCl2, KCl}$ est plus importante que prédite, la piètre résilience des bactériens du sol est d'autant plus conséquente que le pH en puissance est doté d'un vecteur négatif augmentant la probabilité d'une plein réalisation de l'acidité échangeable.

Toutes ces cas de figures sont résumés au Tableau 6.

Enfin, des problèmes de résiliences des populations bactériennes des sols risquent d'apparaître principalement, mais non exclusivement, là où les dpH sont les plus importants. A la Figure 7 apparaissent les cantons pour lesquels les dpH sont en moyenne d'au minimum 0,57, soit la moyenne nationale française (cf. BDAT ; Gissol 2006). A noter que la répartition géographique des ces dpH appréciables n'est pas totalement aléatoire, certains attroupements étant détectables au niveau départemental ; cette agréations des dpH a priori les plus risqués pour la résilience bactérienne dans les sols affectera avantageusement l'application agronomique de l'invention.

**Tableau 6** : Abaque pour l'interprétation des résilience bactériennes, θ, à la lumière des $Pi_{CaCl2}$ (et/ou $Pi_{KCl}$) observées et prédites selon l'équation à la Figure 6. Le risque d'un effet appréciable sur l'activité, la diversité, la tailles des population bactériennes du sol peut ainsi être jaugé, et l'inoculation des résidus de culture pailleux au sol préconisée selon le cas.

| cas de figure | Résilience (θ) PETITE | Résilience (θ) GRANDE |
|---|---|---|
| $Pi_{CaCl2, KCl}$ plus petite que prédite | La bonne résilience des bactéries du sol sera assistée d'un pH en puissance dont le vecteur positif tend à réduire le risque de décrochage de l'activité, la diversité, voire le nombre de bactéries du sol | Malgré une piètre résilience des bactéries du sol, la probabilité qu'elles aient à affronter la plein réalisation de l'acidité échangeable est réduite proportionnellement à la différence entre $Pi_{CaCl2}$ observé et prédit |
| $Pi_{CaCl2, KCl}$ telle que prédite | Pas plus d'information | Pas plus d'information |
| $Pi:_{CaCl2, KCl}$ plus grande que prédit | La bonne résilience des bactéries du sol devra affronter un pH en puissance à la baisse (vecteur négatif) amplifiant la baisse des pH déjà probable dans le cadre de l'acidité échangeable mesuré. | La piètre résilience des bactéries du sol est d'autant plus conséquente que le pH en puissance est doté d'un vecteur négatif augmentant la probabilité d'une plein réalisation de l'acidité échangeable. |

**Références**

Anderson, JPE et Domsch, KH. 1978. A physiological method for the quantitative measurement of microbial biomass in soils. Soil Biology and Biochemistry 10 : 215-221

Arnon, D. 1., Fratzke, W. E. and Johnson, C. M. 1942 Hydrogen ion concentration in relation to absorption of inorganic nutrients by higher plants. Plant Physiol. 17 : 515-524.

Bakken, LR. 1985. Séparation and Purification of Bacteria from Soil. Appl. Environ. Microbiol. 49 : 1482-1487

Bååth, E. 1998. Growth Rates of Bacterial Communities in Soils at Varying pH: A Comparison of the Thymidine and Leucine Incorporation Techniques. Microbial Ecology, Vol. 36, No. 3, pp. 316-327

Baath, E, A Frostegard, T Pennanen et H Fritze. 1995. Microbial community structure and ph response in relation to soil organic matter quality in wood-ash fertilized, clear-cut or burned coniferous forest soils. Soil Biol. Biochem. 21(2): 229-240

Baath, E, A Frostegard et H Fritze. 1992. Soil Bacterial Biomass, Activity, Phospholipid Fatty Acid Pattern, and pH Tolerance in an Area Polluted with Alkaline Dust Déposition. Appl. Environ. Microbiol. 58: 4026-4031

Campbell, CD., SJ. Chapman, CM. Cameron, MS. Davidson et JM. Potts. 2003. A Rapid Microtiter Plate Method To Measure Carbon Dioxide Evolved from Carbon Substrate Amendments so as To Determine the Physiological Profiles of Soil Microbial Communities by Using Whole Soil. Appl. Environ. Microbiol. p. 3593-3599 Vol. 69, No. 6

Claude, P-P. et M. Giroux. 2006. Effet des engrais organo-minéraux inoculés (EOMI) sur la croissance des plants de maïs-grain, les rendements, les prélèvements des éléments nutritifs et la qualité des grains. Agrosolutions 17: 51-64.

Claude, P-P. et L. Fillion. 2004. Effet de l'apport d'un Inoculum bactérien aux résidus de culture de maïs-grain au Sol sur le rendement et la qualité de blés d'hiver panifiables en France. Agrosolutions 15(1) : 23-29.

Devau, N, E Le Cadre, Ph. Hinsinger et F Gérard. 2009. A mechanistic model for understanding pH effect on phosphorus availability in rhizosphere and bulk soil. The Proceedings of the International Plant Nutrition Colloquium XVI, Department of Plant Sciences, US Davis, California. http://www.escholarship.org/uc/item/9nh9b74j. (Publication Date: 08-08-2009).

Dick, WA, L Cheng et P Wang. 2000. Soil acid and alkaline phosphatase activity as pH adjustment indicators. Soil Biol. Biochem. 32 : 1915-1919

Fernandez-Calvino, D. et Erland Baath. 2010. Growth response of the bacterial community to pH in soils differing in pH. FEMS Microbiol Ecol 73 (2010) 149-156

Fierer, N. et RB. Jackson. 2006. The diversity and biogeography of soil bacterial communities. PNAS 103(3) : 626-631

Fierer, N. MA. Bradford et RB. Jackson. Toward an ecological classification of soil bacteria. Ecology 88: 1354-1364

Gissol (Groupement d'intérêt scientifique sol). 2006. Base de Données Analyse des Terres. 1 mars 2006, XP002699913, URL: http://www.gissol.fr/progranrne/bdat/bdat. php

Griffiths, RI. et BC. Thomson, Ph. James, Th. Bell, M. Bailey and AS. Whiteley. 2011. The bacterial biogeography of British soils. Environmental Microbiology 13(6), 1642-1654

Halsall, DM et AH Gibson 1991. Nitrogenase activity in straw-amended wheat belt soils in response to diazotrophe inoculation. Soil Biology and Biochemistry 23(10) : 987-998.

Islam, AKMS, DG Edwards et CJ Asher. 1980. pH Optima For Crop Growth Results Of A Flowing Solution Culture Experiment With Six Species. Plant and Soil 54, 339-357

Jones, DL, D Shannon, D Murphy et J Farrar. 2004. Role of dissolved organic nitrogen (DON) in soil N cycling in grassland soils. Soil Biol. Biochem. 36(5) : 749-756)

Jones, DL et Darrah PR. 1996. Re-Sorption of Organic Compounds By Roots of Zea Mays L. and Its Conséquences In The Rhizosphere. 3. Characteristics Of Sugar Influx And Efflux. Plant and Soil 178: 153-160.

Kemmitt, Sarah J., David Wright et DL. Jones. 2005. Soil acidification used as a management strategy to reduce nitrate losses from agricultural land. Soil Biology & Biochemistry 37 (2005) 867-875

Kemmitt, SJ., D Wright, KWT Goulding et DL. Jones. 2006. pH régulation of carbon and nitrogen dynamics in two agricultural soils. Soil Biology & Biochemistry 38 (2006) 898-911

Kissel, DE, L. Sonon, PF Vendrell et RA Issac. 2009. Salt Concentration and Measurement of Soil pH, Communications in Soil. Science and Plant Analysis, 40:1-6, 179-18

Kleinman, PJA, AN Sharpley, K Gartley, WM Jarrell, S Kuo, RG Menon, R Myers, KR Reddy et EO Skogley. 2001. Interlaboatory comparison of soil phosphorus extacted by various soil test methods. Publications rom USDA-ARS / UNL Faculty. Paper 544. htp://digitalcommons.unl.edu/usdaarsfacpub/544

Koga, K, Y Suehiro, S-T Matsuoka et K Takahashi. 2003. Evaluation of growth activity of microbes in tea field soil using microbila calorimetry. J. Biosci. Bioeng. 95 : 429-434

Koopmans, GF, WJ Chardon, PAI Ehlert, J Dolfing, RAA Suurs, O Oenema et WH van Riemsdijk. 2004. Phosphorus Availability for Plant Uptake in a Phosphorus-Enriched Noncalcareous Sandy Soil. J. Environ. Qual. 33:965-975

Lauber, CL., MS. Strickland, MA. Bradford et N Fierer. 2008. The influence of soil properties on the structure of bacterial and fungal communities across land-use types. Soil Biol. Biochem 40 : 2407-2415

Lauber, CL., Micah Hamady, Rob Knight et Noah Fierer. 2009. Pyrosequencing-Based Assessment of Soil pH as a Predictor of Soil Bacterial Community Structure at the Continental Scale. APPL. ENVIRON. MICROBIOL., Aug. 2009, p. 5111-5120 Vol. 75, No. 15

Lynch, J. et MD Ho. 2005. Rhizoeconomics: Carbon Costs of Phosphorus Acquisition. Plant and Soil 269: 45-56

Miller, RO et DE Kissel. 2010. Comparison of Soil pH Methods on Soils of North America. Soil Sci. Soc. Am. J. 74(1) : 310-316

Nicol, GW. S Leininger, C Schleper et JI. Prosser. 2008. The influence of soil pH on the diversity, abundance and transcriptional activity of ammonia oxidizing archaea and bacteria. Environ. Microbiol. 10: 2966-2978

Ownley BH, DM Weller et LS Thomashow. 1992. Influence of in situ and in vitro pH on suppression of Gaeumannomyces graminis var. tritici by Pseudomonas fluorescens 2-79. Phytopathology 82 : 178-184

Pankratov, TA, YM Serkebaeva, IS Kulichevskaya, W Liesack et SV Dedysh. 2008. Substrate inudced growth and isolation of Acidobacteria from acidic Sphagnum peat. The ISME J. 2 : 551-560.

Parkin, TB., AJ. Sexstone et JM. Tiedje. 1985. Soil pH Adaptation of Denitrifying Populations to Low. Appl. Environ. Microbiol. 1985, 49(5): 1053.

Pautler, MC et JTh Sims. 2000. Relationships Between Soil Test Phosphorus, Soluble Phosphorus, and Phosphorus Saturation in Delaware Soils. Soil Sci. Soc. Am. J. 64:765-773

Peterse, F, GW. Nicol, S Schouten, JS Sinninghe Damsté. 2010. Influence of soil pH on the abundance and distribution of core and intact polar lipid-derived branched GDGTs in soil. Organic. Geochem. 41: 1171-75

Picard, C, C Ponsonnet, E Paget, X Nesme et P. Simonet. 1992. Detectionand Enumeration ofBacteriainSoilby Direct DNA Extractionand Polymerase Chain Reaction. Appl. Environ. Microbiol p.2717-2722 Vol.58,No. 9

Roper MM, RR Gault et NA Smith. 1995. Contribution to the N status of soil by free living nitrogen fixing bacteria in a lucerne stand. Soil Biol. Biochem. 27(4/5) : 467-471

Rosso, L., J R Lobry, S Bajard and J P Flandrois. 1995. Effects of Temperature and pH on Microbial Growth. Convenient Model To Describe the Combined. Appl. Environ. Microbiol. 1995, 61(2):610.

Rousk, Johannes et Davey L. Jones. 2010. Loss of low molecular weight dissolved organic carbon (DOC) and nitrogen (DON) in H2O and 0.5 M K2SO4 soil extracts. Soil Biol. Biochem. 42: 2331-2335

Rousk, J., Philip C. Brookes et Erland Bååth. 2010. The microbial PLFA composition as affected by pH in an arable soil. Soil Biology & Biochemistry 42 (2010) 516-520

Rousk, J., PC. Brookes et E Bååth. 2010. Investigating the mechanisms for the opposing pH relationships of fungal and bacterial growth in soil. Soil Biology & Biochemistry 42 (2010) 926e934

Rousk, J, PC. Brookes et E Bååth. 2009. Contrasting Soil pH Effects on Fungal and Bacterial Growth Suggest Functional Redundancy in Carbon Mineralization. Appl. Environ. Microbiol., Mar. 2009, p. 1589-1596 Vol. 75, No. 6

Rousk, J., PC. Brookes, HC. Glanville et DL. Jones. 2011. Lack of Correlation between Turnover of Low-Molecular-Weight Dissolved Organic Carbon and Differences in Microbial Community Composition or Growth across a Soil pH Gradient. Appl. Environ. Microbiol., p. 2791-2795 Vol. 77, No. 8

Rousk, J., E Baath, PC Brookes, CL Lauber, C Lozupone, JG Caporaso, R Knight et N Fierer. 2010. Soil bacterial and fungal communities across a pH gradient in an arable soil. ISME Journal 4: 1340-1351

Sessitsch, A., E. Hackl, P. Wenzl, A. Kilian, T. Kostic, N. Stralis-Pavese, B. Tankouo Sandjong et L. Bodrossy. 2006. Diagnostic microbial microarrays in soil ecology. New Phytologist 171 : 719-736.

Šimek, M. et D.W. Hopkins. 1999. Regulation of potential denitrification by soil pH in long-term fertilized arable soils. Biol Fertil Soils (1999) 30 :41-47

Simek, M., L Jisova, DW. Hopkins. 2002. What is the so-called optimum pH for denitrification in soil? Soil Biol Biochem. 34: 1227-1234

Taziebou, LC, FX Etoa et JJ Essia Ngang. 2004. Effects of some environmental factors on microbial growth and biodegradability of a pesticide mixture by soil microorganimsms. J. Camer. Acad. Sci. 4:41-47

Wakelin, SA., L.M.Macdonald, S.L.Rogers , A.L.Gregga, T.P.Bolger et J.A.Baldock. 2008. Habitat selective factors influencing the structural composition and functional capacity of microbial communities in agricultural soils. Soil Biol. Biochem. 40: 803-813

Wonnacott, TH et RJ Wonnacott. 1991 (traduction française). Statistique - économie, gestion, sciences, médecine (4ième édition). Economia, 49, rue Héricart, 75015 Paris.

Wu, U., B Ma, L Zhou, H Wang, J Xu, S Kemmitt et PC. Brookes. Changes in the soil microbial community structure with latitude in eastern China, based on phospholipid fatty acid analysis. Appl. Soil Ecol. 43: 234-240

## Revendications

1. Méthode d'évaluation de la résilience de la fraction bactérienne de la microflore d'un sol arable ou forestier en termes de diversité, d'activité et/ou d'abondance confrontée à des variations de pH du sol environnant comprenant les étapes suivantes ;

   • **échantillonnage** du sol d'une parcelle agronomique et/ou d'îlots agricoles, ou encore d'une parcelle forestière,
   • **mesure** de la dynamique de la fraction bactérienne du sol en termes de diversité, d'activité et/ou d'abondance, relative ou absolue, à différentes valeurs de pH comprises à l'intérieure d'une plage de valeurs correspondant à l'étendue de l'acidité échangeable du sol,
   • **comparaison** de cette mesure avec des valeurs optimales permettant de constater un écart significatif,
   • **diagnostique** de l'état microbiologique et/ou bactériologique du sol par appréciation de cet écart permettant de préconiser une pratique agronomique capable de renforcer ces populations bactériennes,

   **caractérisée en ce que** la mesure de la résilience est obtenue par superposition de la courbe décrivant cette variation de la dynamique de ladite fraction bactérienne de la microflore du sol en fonction des susdites différentes valeurs de pH imposées *in vitro* (courbe variable), à une autre courbe fixée horizontalement (courbe fixe) à partir d'un pH optimal ($pH_o$) et/ou supposé optimal pour ladite dynamique bactérienne en termes de diversité, d'activité et/ou d'abondance, relative ou absolue.

2. Méthode d'évaluation de la résilience de la fraction bactérienne de la microflore d'un sol selon la première revendication **caractérisée en ce que** ledit $pH_o$ dans le sol est soit le $pH_{eau}$, soit le pH maximal effectivement observé à travers une gamme de milieux de culture au pH tamponnés à diverses valeurs permettant ainsi d'observer une augmentation puis une diminution de la dynamique de la fraction bactérienne de la microflore du sol en terme de diversité, d'activité et/ou d'abondance, relative ou absolue.

3. Méthode d'évaluation de la résilience de la fraction bactérienne de la microflore d'un sol selon une quelconque des revendications précédentes **caractérisée en ce que** la plage de valeurs correspondant à l'étendue de l'acidité échangeable du sol est établie par titrage incrémental de l'acidité non résiduelle du sol, et/ou, plus simplement par la différence entre les $pH_{eau}$ et $pH_{KCl}$ ou $pH_{CaCl2}$, ce dernier cas de figure ne donnant comme de raison que deux (2) points pour établir lesdites courbes fixes et variable.

4. Méthode d'évaluation de la résilience de la fraction bactérienne de la microflore d'un sol selon une quelconque des revendications 1 à 3 précédentes **caractérisée en ce que** la mesure de la dynamique de la fraction bactérienne de la microflore du sol se fait sur la base du métabolisme d'un substrat carboné à différentes valeurs de pH comprises à l'intérieur d'une plage de valeurs correspondant à l'étendue de l'acidité échangeable.

5. Méthode d'évaluation de la résilience de la fraction bactérienne de la microflore d'un sol selon une quelconque des revendications 1 à 3 précédentes **caractérisée en ce que** la mesure de la dynamique de la fraction bactérienne de la microflore du sol se fait sur la base du dénombrement de cellules bactériennes viables à différentes valeurs de pH comprises à l'intérieure d'une plage de valeurs correspondant à l'étendue de l'acidité échangeable.

6. Méthode d'évaluation de la résilience de la fraction bactérienne de la microflore d'un sol selon une quelconque des revendications 1 à 3 précédentes **caractérisée en ce que** la mesure de la dynamique de la fraction bactérienne de la microflore du sol ce fait sur la base d'un indice de diversité de la microflore bactérienne à différentes valeurs de pH comprises à l'intérieure d'un plage de valeurs correspondant à l'étendue de l'acidité échangeable

7. Méthode d'évaluation de la résilience de la fraction bactérienne de la microflore d'un sol selon une quelconque des revendications précédentes **caractérisée en ce que** la comparaison de la mesure de la dynamique bactérienne par superposition des courbes, fixes et variables, comprend une intervalle de confiance applicable à ces deux courbes.

8. Méthode d'évaluation de la résilience de la fraction bactérienne de la microflore d'un sol selon une quelconque des revendications précédentes **caractérisée en ce que** la comparaison de la mesure de la dynamique bactérienne par superposition des courbes fixes et variables comprend un intervalle de confiance applicable à ces deux courbes au-delà de laquelle la courbe variable s'écarte significativement de la courbe fixe.

9. Méthode d'évaluation de la résilience de la fraction bactérienne de la microflore d'un sol selon une quelconque des revendications précédentes **caractérisée en ce que** lorsque la courbe dite variable s'écarte significativement de la courbe fixe, *i.e.* au-delà des bornes établies par les intervalles de confiances, il est possible de graduer ce manque de résilience en mesurant l'angle, $\theta$, entre les courbes variables et fixes.

10. Méthode d'évaluation de la résilience de la fraction bactérienne de la microflore d'un sol selon la revendication précédente **caractérisée en ce que** si la courbe dite variable s'écarte significativement de la courbe fixe, *i.e.* au-delà des bornes établies par les intervalles de confiances en raison d'un angle, $\theta$, important entre ces deux courbes, les populations bactériennes en question sont dites peu ou pas résilientes, et le renforcement de ces populations, par inoculation bactérienne et/ou azotobactérienne par exemple, est *préconisable.*

11. Méthode d'évaluation de la résilience de la fraction bactérienne de la microflore d'un sol selon une quelconque des revendications précédentes **caractérisée en ce que** le diagnostique de l'état microbiologique et/ou bactériologique sur la base de l'angle $\theta$, mesure de la résilience des populations bactériennes de l'échantillon du sol, est pondéré à l'aide d'une mesure du phosphore inorganique, Pi, en solution d'extraction avant et après le titrage de l'acidité échangeable du sol.

12. Méthode d'évaluation de la résilience de la fraction bactérienne de la microflore d'un sol selon la revendication précédente **caractérisée en ce que** qu'il existe une relation entre la teneur en Pi en solution d'extraction de l'échantillon avant et après titrage de l'acidité échangeable.

13. Méthode d'évaluation de la résilience de la fraction bactérienne de la microflore d'un sol selon les revendications 11 et 12 **caractérisée en ce que** ladite relation entre la teneur en Pi de l'échantillon avant et après titrage de l'acidité échangeable est linéaire et avantageusement décrite par une simple régression linéaire dont la pente est comprise entre des valeurs de 0,50 à 0,25, plus particulièrement de 0,40 à 0,30, et avantageusement de 0,38 +/- 0,05.

14. Méthode d'évaluation de la résilience de la fraction bactérienne de la microflore d'un sol selon les revendications 11 à 13 **caractérisée en ce que** la pondération du diagnostique de l'état microbiologique et/ou bactériologique sur la base de l'angle $\theta$, mesure de la résilience des populations bactériennes de l'échantillon du sol est effectué comme suit ;

> si le $Pi_{CaCl2, KCl}$ observé est supérieur à celui prédit à partir d'une relation [$Pi_{CaCl, KCl}$ : $Pi_{eau}$], et sachant que $Pi_{CaCl2, KCl} < Pi_{eau}$, le pH effectif est probablement plus grand que le $pH_{CaCl2, KCl}$, ce vecteur va atténuer la pleine réalisation de l'acidité échangeable,

> si le $Pi_{CaCl2, KCl}$ observé est égale à celui prédit et aucune information nouvelle est apportée,

> si le $Pi_{CaCl2, KCl}$ observé est inférieur à celui prédit, et sachant encore une fois que $Pi_{CaCl2, KCl} < Pi_{eau}$, le pH effectif, en puissance en sorte, est vraisemblablement plus faible que le $pH_{CaCl2}$ ou $pH_{KCl}$, observé, ce vecteur va accentuer la pleine réalisation de l'acidité échangeable,

de manière à ce que en combinaison avec les mesures de θ, il est donc possible d'établir un diagnostique de l'état bactériologique du sol pour les situations suivante ;

> lorsque θ est faible, et si $Pi_{CaCl2, KCl}$ est moins importante que prédite, la bonne résilience des bactéries du sol sera assistée d'un pH plus haut en puissance qui tend à réduire le risque de décrochage de ngaBAC. Si $Pi_{CaCl2, KCl}$ est plus importante que prédite, la bonne résilience des bactéries du sol devra affronter un pH plus bas en puissance amplifiant ainsi la baisse des pH déjà probable dans le cadre de l'acidité échangeable mesuré.

> lorsque θ est importante, et si $Pi_{CaCl2, KCl}$ est moins importante que prédite, malgré une piètre résilience des bactéries du sol, la probabilité qu'elles aient à affronter la plein réalisation de l'acidité échangeable est réduite proportionnellement à la différence entre $Pi_{CaCl2, KCl}$ observée et prédite. Si $Pi_{CaCl2, KCl}$ est plus importante que prédite, la piètre résilience des bactéries du sol est d'autant plus conséquente que le pH plus bas en puissance va augmenter la probabilité d'une plein réalisation de l'acidité échangeable du sol.

## Patentansprüche

1. Methode zur Bewertung der Widerstandsfähigkeit der bakteriellen Fraktion der Mikroflora von Acker- oder Waldboden hinsichtlich Vielfalt, Aktivität und/oder Häufigkeit gegenüber pH-Wert-Schwankungen des umgebenden Bodens, die die folgenden Schritte umfasst:

   • **Beprobung** der Böden landwirtschaftlicher Parzellen und/oder Blöcke oder Waldgrundstücke,
   • **Messung** der Dynamik der Fraktion der Bodenbakterien Hinblick auf relative oder absolute Diversität, Aktivität und/oder Häufigkeit bei verschiedenen pH-Werten innerhalb eines Wertebereichs, der dem Ausmaß der austauschbaren Bodenacidität entspricht,
   • **Vergleich** dieser Messung mit optimalen Werten, um einen signifikanten Unterschied feststellen zu können,
   • **Diagnose** des mikrobiologischen und/oder bakteriologischen Bodenzustands durch Einschätzung dieser Diskrepanz, um eine landwirtschaftliche Praxis zur Stärkung dieser Bakterienpopulationen empfehlen zu können,

   und dadurch charakterisiert ist, dass die Messung der Widerstandsfähigkeit erhalten wird, indem die Kurve, die diese Veränderung in der Dynamik der bakteriellen Fraktion der Bodenmikroflora als Funktion der verschiedenen in vitro erhobenen pH-Werte (variable Kurve) beschreibt, mit einer anderen horizontalen (festen Kurve) für einen vermeintlich optimalen pH-Wert (pHo) übereinandergelegt wird, für die bakterielle Dynamik in Bezug auf Vielfalt, Aktivität und/oder Häufigkeit, relativ oder absolut, angenommen wird.

2. Methode zur Bewertung der Widerstandsfähigkeit der bakteriellen Fraktion der Bodenmikroflora gemäß dem ersten Anspruch, die dadurch charakterisiert ist, dass der pHo im Boden entweder dem $pH_{Wasser}$ oder dem maximalen pH-Wert entspricht, der tatsächlich in einem Bereich von pHgepufferten Kulturmedien bei verschiedenen Werten beobachtet wird, wodurch es möglich ist, eine Zunahme und dann eine Abnahme der Dynamik der bakteriellen Fraktion der Bodenmikroflora in Bezug auf Vielfalt, Aktivität und/oder Häufigkeit, relativ oder absolut, zu beobachten.

3. Methode zur Bewertung der Widerstandsfähigkeit der bakteriellen Fraktion der Bodenmikroflora gemäß einem der vorhergehenden Ansprüche, die dadurch charakterisiert ist, dass der Wertebereich, der dem Ausmaß der austauschbaren Säure des Bodens entspricht, durch inkrementelle Titration der nicht-restlichen Säure des Bodens und/oder, einfacher gesagt, durch die Differenz zwischen $pH_{Wasser}$ und $pH_{KCl}$ oder $pH_{CaCl2}$ bestimmt wird, wobei letzterer Fall nur zwei (2) Punkte zur Festlegung der festen und variablen Kurven ergibt.

4. Methode zur Bewertung der Widerstandsfähigkeit der bakteriellen Fraktion der Bodenmikroflora gemäß einem der vorstehenden Ansprüche 1 bis 3, die dadurch charakterisiert ist, dass die Messung der Dynamik der bakteriellen Fraktion der Bodenmikroflora auf der Grundlage des Stoffwechsels eines Kohlenstoffsubstrats bei unterschiedlichen pH-Werten innerhalb eines Wertebereichs durchgeführt wird, der dem Ausmaß der austauschbaren Säure entspricht.

5. Methode zur Bewertung der Widerstandsfähigkeit der bakteriellen Fraktion der Bodenmikroflora gemäß einem der vorstehenden Ansprüche 1 bis 3, die dadurch charakterisiert ist, dass die Messung der Dynamik der bakteriellen Fraktion der Bodenmikroflora auf der Grundlage der Zählung lebensfähiger Bakterienzellen bei unterschiedlichen pH-Werten innerhalb eines Wertebereichs durchgeführt wird, der dem Bereich der austauschbaren Säure entspricht.

6. Methode zur Bewertung der Widerstandsfähigkeit der bakteriellen Fraktion der Bodenmikroflora gemäß einem der vorstehenden Ansprüche 1 bis 3, die dadurch charakterisiert ist, dass die Messung der Dynamik der bakteriellen Fraktion der Bodenmikroflora auf der Grundlage eines Diversitäts-Index der bakteriellen Mikroflora bei unterschiedlichen pH-Werten innerhalb eines Wertebereichs durchgeführt wird, der dem Bereich der austauschbaren Säure entspricht.

7. Methode zur Bewertung der Widerstandsfähigkeit der bakteriellen Fraktion der Bodenmikroflora gemäß einem der vorstehenden Ansprüche, die dadurch charakterisiert ist, dass der Vergleich der Messung der bakteriellen Dynamik durch Übereinanderlegen der festen und variablen Kurven ein auf diese beiden Kurven anwendbares Konfidenzintervall umfasst.

8. Methode zur Bewertung der Widerstandsfähigkeit der bakteriellen Fraktion der Bodenmikroflora gemäß einem der vorstehenden Ansprüche, die dadurch charakterisiert ist, dass der Vergleich der Messung der bakteriellen Dynamik durch Übereinanderlegen der festen und variablen Kurven ein für diese beiden Kurven anwendbares Konfidenzintervall umfasst, durch das die variable Kurve signifikant von der festen Kurve abweicht.

9. Methode zur Bewertung der Widerstandsfähigkeit der bakteriellen Fraktion der Bodenmikroflora gemäß einem der vorstehenden Ansprüche, die dadurch charakterisiert ist, dass wenn die sogenannte variable Kurve signifikant von der fixen Kurve abweicht, d. h. über die durch die Konfidenzintervalle festgelegten Grenzen hinaus, dieser Mangel an Widerstandsfähigkeit durch Messung des Winkels $\theta$ zwischen der variablen und der festen Kurve skaliert werden kann.

10. Methode zur Bewertung der Widerstandsfähigkeit der bakteriellen Fraktion der Bodenmikroflora gemäß dem vorstehenden Anspruch, die dadurch charakterisiert ist, dass, wenn die sogenannte variable Kurve signifikant von der fixen Kurve abweicht, d. h. über die durch die Konfidenzintervalle aufgrund eines Winkels $\theta$, der für die beiden Kurven wichtig ist, festgelegten Grenzen hinaus, die betreffenden Bakterienpopulationen als wenig oder nicht belastbar gelten und die Verstärkung dieser Populationen, beispielsweise durch bakterielle und/oder azotobakterielle Inokulation, empfehlenswert ist.

11. Methode zur Bewertung der Widerstandsfähigkeit der bakteriellen Fraktion der Bodenmikroflora gemäß einem der vorstehenden Ansprüche, die dadurch charakterisiert ist, dass die Diagnose des mikrobiologischen und/oder bakteriologischen Zustands auf Grundlage des Winkels $\theta$, Messung der Widerstandsfähigkeit der Bakterienpopulationen der Bodenprobe unter Verwendung einer Messung von anorganischem Phosphor, Pi, in Extraktionslösung vor und nach der Titration der austauschbaren Säure des Bodens gewichtet wird.

12. Methode zur Bewertung der Widerstandsfähigkeit der bakteriellen Fraktion der Bodenmikroflora gemäß dem vorstehenden Anspruch, die dadurch charakterisiert ist, dass eine Beziehung zwischen dem Pi-Gehalt in der Extraktionslösung der Probe vor und nach der Titration der austauschbaren Säure besteht.

13. Methode zur Bewertung der Widerstandsfähigkeit der bakteriellen Fraktion der Bodenmikroflora gemäß den Ansprüchen 11 und 12, die dadurch charakterisiert ist, dass die besagte Beziehung zwischen dem Pi-Gehalt der Probe vor und nach der Titration der austauschbaren Säure linear und vorteilhaft durch eine einfache lineare Regression beschrieben wird, deren Steigung zwischen Werten von 0,50 bis 0,25, insbesondere zwischen 0,40 und 0,30, und im günstigen Fall im Bereich 0,38 +/- 0,05 liegt.

14. Methode zur Bewertung der Widerstandsfähigkeit der bakteriellen Fraktion der Bodenmikroflora gemäß den Ansprüchen 11 bis 13, die dadurch charakterisiert ist, dass die diagnostische Gewichtung des mikrobiologischen und/oder bakteriologischen Zustands anhand des Winkels $\theta$, Maß für die Widerstandsfähigkeit der Bakterienpopulationen der Bodenprobe, wie folgt durchgeführt wird:

➢ wenn der beobachtete $Pi_{CaCl2,\ KCl}$ höher ist als der aus einer Relation vorhergesagte [$Pic_{CaCl,\ KCl}$: $Pi_{Wasser}$], und wissend, dass $Pi_{CaCl2,\ KCl} < Pi_{Wasser}$, ist der effektive pH-Wert wahrscheinlich größer ist als $pH_{CaCl2,\ KCl}$, wird dieser Vektor das volle Erreichen der austauschbaren Säure abschwächen,

➢ wenn der beobachtete $Pi_{CaCl2,\ KCl}$ dem vorhergesagten entspricht und keine neuen Informationen geliefert werden,

➢ wenn der beobachtete $Pi_{CaCl2,\ KCl}$ niedriger ist als vorhergesagte, und wieder wissend, dass $Pi_{CaCl2,\ KCl} <$ Piwasser, der effektive pH-Wert in der Stärke wahrscheinlich niedriger ist als der beobachtete $pH_{CaCl2}$ oder $pH_{KCl}$, wird dieser Vektor die volle Realisierung des austauschbaren Säuregehalts betonen,

sodass in Kombination mit den Messungen von θ eine Diagnose des bakteriologischen Zustands des Bodens für folgende Situationen möglich ist;

➢ wenn θ niedrig ist, und wenn $Pi_{CaCl2,\ KCl}$ weniger wichtig ist als vorhergesagt, wird die gute Widerstandsfähigkeit der Bodenbakterien durch einen höheren pH-Wert unterstützt, was das Risiko eines Abnehmens von ngaBAC verringert. Wenn $Pi_{CaCl2,\ KCl}$ wichtiger ist als vorhergesagt, wird die gute Widerstandsfähigkeit der Bodenbakterien mit dem niedrigsten pH-Wert konfrontiert werden, wodurch die bereits im Rahmen der gemessenen austauschbaren Säure wahrscheinliche Abnahme des pH-Wertes verstärkt wird.

➢ wenn θ wichtig ist, und wenn $Pi_{CaCl2,\ KCl}$ trotz schlechter Widerstandsfähigkeit der Bodenbakterien weniger wichtig ist als vorhergesagt, wird die Wahrscheinlichkeit, dass sie mit der vollen Realisierung der austauschbaren Säure konfrontiert werden, proportional zur Differenz zwischen dem beobachteten und dem vorhergesagten $Pi_{CaCl2,\ KCl}$ reduziert. Wenn $Pi_{CaCl2,\ KCl}$ wichtiger ist als vorhergesagt, ist die schlechte Widerstandsfähigkeit der Bodenbakterien umso bedeutender, da der niedrigste pH-Wert die Wahrscheinlichkeit einer vollständigen Umsetzung der austauschbaren Säure des Bodens erhöht.

## Claims

1. Method for assessing the resilience of the bacterial fraction of the microflora of arable or forest soil in terms of diversity, activity and/or abundance when confronted with variations in the pH of the surrounding soil comprised of;

   • soil **sampling** of agricultural plot and/or islets or of a forest plot,
   • **measuring** the dynamics of the bacterial fraction of the soil in terms of diversity, activity and/or abundance, relative or absolute, at different pH values in the range of values corresponding to the extent the exchangeable acidity of the soil,
   • **comparison** of this measure with optimal values making it possible to observe a significant difference,
   • microbiological and/or bacteriological **diagnosis** of the soil by assessing the aforesaid difference allowing for the recommendation of an agronomic practice that can strengthen these bacterial populations,

   **characterized in that** the measure of resilience is obtained by superposition of the curve describing the variation of the dynamics of said bacterial fraction of soil microflora according to the aforesaid different pH values *in vitro* (variable curve) onto another fixed horizontal curved fixed (fixed curve) at an optimal pH (pHo) and/or supposed optimal for the aforesaid bacterial dynamics in terms of diversity, activity and/or abundance, relative or absolute.

2. Method for evaluating the resilience of the bacterial fraction of the microflora of a soil according to the first claim, **characterized in that** said pHo in the soil is either the $pH_{water}$ or the maximum pH observed across a range of culture media buffered at various pH values allowing for an increase and then a decrease in the dynamics of the bacterial fraction of the soil microflora in terms of diversity, activity and/or abundance, relative or absolute.

3. Method for evaluating the resilience of the bacterial fraction of the microflora of a soil according to any one of the preceding claims, **characterized in that** the range of pH values corresponding to the extent of the exchangeable acidity of the soil is established by incremental titration of the non-residual acidity of the soil and/or, more simply, by the difference between the $pH_{water}$ and $pH_{KCl}$ or $pH_{CaCl2}$, the latter yield of coarse only two (2) points to establish the said curves, fixed and variable.

4. Method for evaluating the resilience of the bacterial fraction of the microflora of a soil according to any one of the preceding claims 1 to 3 **characterized in that** the measurement of the dynamics of the bacterial fraction of soil microflora is made on the basis of the metabolism of a carbon substrate at different pH values within the range of pH values corresponding to the extent of the exchangeable acidity.

5. Method for evaluating the resilience of the bacterial fraction of the microflora of a soil according to any one of the preceding claims 1 to 3 **characterized in that** the measurement dynamics of the bacterial fraction of soil microflora is made on the basis of the enumeration of viable bacterial cells at different pH values within the range of pH values corresponding to the extent of the exchangeable acidity.

6. Method for evaluating the resilience of the bacterial fraction of the microflora of a soil according to any one of the preceding claims 1 to 3 **characterized in that** the measurement dynamics of the bacterial fraction of soil microflora is made on the basis of a diversity index of the bacterial microflora at different pH values within a range of pH values corresponding to the extent of exchangeable acidity.

7. Method for evaluating the resilience of the bacterial fraction of the microflora of a soil according to any of the preceding claims **characterized in that** the comparison of the bacterial dynamics made by superimposing the aforesaid fixed and variable curves comprises a confidence interval applicable to these two curves.

8. Method for evaluating the resilience of the bacterial fraction of the microflora of a soil according to any of the preceding claims **characterized in that** the comparison of the measurement of the bacterial dynamics by superimposing the aforesaid fixed and variable curves comprises a confidence interval applicable to these two curves beyond which the variable curve deviates significantly from the fixed curve.

9. Method for evaluating the resilience of the bacterial fraction of the microflora of a soil according to any one of the preceding claims **characterized in that** when the so-called variable curve deviates significantly from the fixed curve, *i.e.* beyond the limits established by the said confidence intervals, it is possible to grade this lack of resilience by measuring the angle, $\theta$, between the variable and fixed curves.

10. Method for evaluating the resilience of the bacterial fraction of the microflora of a soil according to the preceding claim **characterized in that** if the so-called variable curve deviates significantly from the fixed curve , *i.e.* beyond the limits established by the confidence intervals due to an significant angle, $\theta$, between these two curves, the bacterial populations in question are said to be poorly or not resilient, and strengthening of these populations, by bacterial and/or azotobacterial inoculation for example, is advocated.

11. Method for evaluating the resilience of the bacterial fraction of the microflora of a soil according to any one of the preceding claims, **characterized in that** the diagnosis of the microbiological and/or bacteriological state on the basis of the angle $\theta$, a measure of the resilience of the bacterial populations of the soil sample, is weighted using a measurement of the inorganic phosphorus, Pi, in extraction solution before and after titration of the exchangeable soil acidity.

12. Method for evaluating the resilience of the bacterial fraction of the microflora of a soil according to the preceding claim **characterized in that** there is a relationship between the content of Pi in the extraction solution of the sample before and after titration exchangeable acidity.

13. Method for evaluating the resilience of the bacterial fraction of the microflora of a soil according to claims 11 and 12, **characterized in that** the said relationship between the Pi content of the sample before and after titration of the exchangeable acidity is linear and advantageously described by a simple linear regression whose slope is between values of 0.50 to 0.25, more particularly of 0.40 to 0.30, and advantageously of 0.38 +/- 0,05.

14. Method for evaluating the resilience of the bacterial fraction of the microflora of a soil according to claims 11-1 3, **characterized in that** the weighting of the diagnosis microbiological state and/or bacteriological based on the angle $\theta$, a measure of the resilience of the bacterial populations of the soil sample is performed as follows;

> if the observed $Pi_{CaCl2,\ KCl}$ is greater than the predicted from a relationship [$Pi_{CaCl,\ KCl}$: $Pi_{water}$], and knowing that $Pi_{CaCl2,\ KCl} < Pi_{water}$, the effective pH is probably greater than the $pH_{CaCl2,\ KCl}$, this vector will mitigate the full attainment of exchangeable acidity,

> if the observed $Pi_{CaCl2, KCl}$ is equal to that predicted and no new information is provided,
> if the observed $Pi_{CaCl2, KCl}$ smaller than that predicted, and knowing again that $Pi_{CaCl2, KCl} < Pi_{water}$, the effective pH is likely lower than the observed $pH_{CaCl2}$ or $pH_{KCl}$ and this vector will accentuate the full attainment of the exchangeable acid,

so that in combination with the measurements of $\theta$, it is therefore possible to establish a diagnosis of the bacteriological state of the soil for the following situations;

> when $\theta$ is small, and if $Pi_{CaCl2, KCl}$ is less than predicted, the good resilience of soil bacteria will be assisted by a higher pH that tends to reduce the risk of a drop in ngaBAC. If $Pi_{CaCl2, KCl}$ is larger than predicted, the good resilience of the soil bacteria will have to face a lower pH in power, thus amplifying the drop in pH already probable in the context of the measured exchangeable acidity.
> when $\theta$ is large, and if $Pi_{CaCl2, KCl}$ is less important than predicted, despite a poor resilience of soil bacteria, the probability that they will have to deal with the full realization of exchangeable acidity is reduced in proportion to the difference between the observed and predicted $Pi_{CaCl2, KCl}$. If $Pi_{CaCl2, KCl}$ is larger than predicted, the poor resilience of soil bacteria is all the more important as the lower pH in power will increase the probability of full deployment of exchangeable soil acidity.

Figure 1

Figure 2

Figure 3

Figure 4

Figure 5

Figure 6

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2314669 A
- FR 1201990
- EP 13366004 A
- EP 2213154 A


**Littérature non-brevet citée dans la description**

- **ANDERSON, JPE ; DOMSCH, KH.** A physiological method for the quantitative measurement of microbial biomass in soils. *Soil Biology and Biochemistry,* 1978, vol. 10, 215-221
- **ARNON, D. 1. ; FRATZKE, W. E. ; JOHNSON, C. M.** Hydrogen ion concentration in relation to absorption of inorganic nutrients by higher plants. *Plant Physiol.,* 1942, vol. 17, 515-524
- **BAKKEN, LR.** Séparation and Purification of Bacteria from Soil. *Appl. Environ. Microbiol.,* 1985, vol. 49, 1482-1487
- **BÅÅTH, E.** Growth Rates of Bacterial Communities in Soils at Varying pH: A Comparison of the Thymidine and Leucine Incorporation Techniques. *Microbial Ecology,* 1998, vol. 36 (3), 316-327
- **BAATH, E ; A FROSTEGARD ; T PENNANEN ; H FRITZE.** Microbial community structure and ph response in relation to soil organic matter quality in wood-ash fertilized, clear-cut or burned coniferous forest soils. *Soil Biol. Biochem.,* 1995, vol. 21 (2), 229-240
- **BAATH, E ; A FROSTEGARD ; H FRITZE.** Soil Bacterial Biomass, Activity, Phospholipid Fatty Acid Pattern, and pH Tolerance in an Area Polluted with Alkaline Dust Déposition. *Appl. Environ. Microbiol.,* 1992, vol. 58, 4026-4031
- **CAMPBELL, CD. ; SJ. CHAPMAN ; CM. CAMERON ; MS. DAVIDSON ; JM. POTTS.** A Rapid Microtiter Plate Method To Measure Carbon Dioxide Evolved from Carbon Substrate Amendments so as To Determine the Physiological Profiles of Soil Microbial Communities by Using Whole. *Soil. Appl. Environ. Microbiol.,* 2003, vol. 69 (6), 3593-3599
- **CLAUDE, P-P. ; M. GIROUX.** Effet des engrais organo-minéraux inoculés (EOMI) sur la croissance des plants de maïs-grain, les rendements, les prélèvements des éléments nutritifs et la qualité des grains. *Agrosolutions,* 2006, vol. 17, 51-64
- **CLAUDE, P-P. ; L. FILLION.** Effet de l'apport d'un Inoculum bactérien aux résidus de culture de maïs-grain au Sol sur le rendement et la qualité de blés d'hiver panifiables en France. *Agrosolutions,* 2004, vol. 15 (1), 23-29
- A mechanistic model for understanding pH effect on phosphorus availability in rhizosphere and bulk soil. **DEVAU, N ; E LE CADRE ; PH. HINSINGER ; F GÉRARD.** The Proceedings of the International Plant Nutrition Colloquium. Department of Plant Sciences, 08 Août 2009, vol. XVI
- **DICK, WA ; L CHENG ; P WANG.** Soil acid and alkaline phosphatase activity as pH adjustment indicators. *Soil Biol. Biochem.,* 2000, vol. 32, 1915-1919
- **FERNANDEZ-CALVINO, D. ; ERLAND BAATH.** Growth response of the bacterial community to pH in soils differing in pH. *FEMS Microbiol Ecol,* 2010, vol. 73, 149-156
- **FIERER, N. ; RB. JACKSON.** The diversity and biogeography of soil bacterial communities. *PNAS,* 2006, vol. 103 (3), 626-631
- **FIERER, N. ; MA. BRADFORD ; RB. JACKSON.** Toward an ecological classification of soil bacteria. *Ecology,* vol. 88, 1354-1364
- **GISSOL.** Groupement d'intérêt scientifique sol. *Base de Données Analyse des Terres,* 01 Mars 2006, http://www.gissol.fr/progranrne/bdat/bdat. php
- **GRIFFITHS, RI. ; BC. THOMSON ; PH. JAMES ; TH. BELL ; M. BAILEY ; AS. WHITELEY.** The bacterial biogeography of British soils. *Environmental Microbiology,* 2011, vol. 13 (6), 1642-1654
- **HALSALL, DM ; AH GIBSON.** Nitrogenase activity in straw-amended wheat belt soils in response to diazotrophe inoculation. *Soil Biology and Biochemistry,* 1991, vol. 23 (10), 987-998
- **ISLAM ; AKMS ; DG EDWARDS ; CJ ASHER.** pH Optima For Crop Growth Results Of A Flowing Solution Culture Experiment With Six Species. *Plant and Soil,* 1980, vol. 54, 339-357
- **JONES, DL ; D SHANNON ; D MURPHY ; J FARRAR.** Role of dissolved organic nitrogen (DON) in soil N cycling in grassland soils. *Soil Biol. Biochem.,* 2004, vol. 36 (5), 749-756

- **JONES, DL ; DARRAH PR.** Re-Sorption of Organic Compounds By Roots of Zea Mays L. and Its Conséquences. *The Rhizosphere. 3. Characteristics Of Sugar Influx And Efflux. Plant and Soil,* 1996, vol. 178, 153-160
- **KEMMITT ; SARAH J. ; DAVID WRIGHT ; DL. JONES.** Soil acidification used as a management strategy to reduce nitrate losses from agricultural land. *Soil Biology & Biochemistry,* 2005, vol. 37, 867-875
- **KEMMITT, SJ. ; D WRIGHT ; KWT GOULDING ; DL. JONES.** pH régulation of carbon and nitrogen dynamics in two agricultural soils. *Soil Biology & Biochemistry,* 2006, vol. 38, 898-911
- **KISSEL, DE ; L. SONON ; PF VENDRELL ; RA ISSAC.** Salt Concentration and Measurement of Soil pH, Communications in Soil. *Science and Plant Analysis,* 2009, vol. 40 (1-6), 179-18
- Interlaboatory comparison of soil phosphorus extacted. **KLEINMAN, PJA ; AN SHARPLEY ; K GARTLEY ; WM JARRELL ; S KUO ; RG MENON ; R MYERS ; KR REDDY ; EO SKOGLEY.** various soil test methods. USDA-ARS / UNL Faculty, 2001
- **KOGA, K ; Y SUEHIRO ; S-T MATSUOKA ; K TAKAHASHI.** Evaluation of growth activity of microbes in tea field soil using microbila calorimetry. *J. Biosci. Bioeng.,* 2003, vol. 95, 429-434
- **KOOPMANS, GF ; WJ CHARDON ; PAI EHLERT ; J DOLFING ; RAA SUURS ; O OENEMA ; WH VAN RIEMSDIJK.** Phosphorus Availability for Plant Uptake in a Phosphorus-Enriched Noncalcareous Sandy Soil. *J. Environ. Qual.,* 2004, vol. 33, 965-975
- **LAUBER, CL. ; MS. STRICKLAND ; MA. BRADFORD ; N FIERER.** The influence of soil properties on the structure of bacterial and fungal communities across land-use types. *Soil Biol. Biochem,* 2008, vol. 40, 2407-2415
- **LAUBER, CL. ; MICAH HAMADY ; ROB KNIGHT ; NOAH FIERER.** Pyrosequencing-Based Assessment of Soil pH as a Predictor of Soil Bacterial Community Structure at the Continental Scale. *APPL. ENVIRON. MICROBIOL.,* Août 2009, vol. 75 (15), 5111-5120
- **LYNCH, J. ; MD HO.** Rhizoeconomics: Carbon Costs of Phosphorus Acquisition. *Plant and Soil,* 2005, vol. 269, 45-56
- **MILLER, RO ; DE KISSEL.** Comparison of Soil pH Methods on Soils of North America. *Soil Sci. Soc. Am. J.,* 2010, vol. 74 (1), 310-316
- **NICOL, GW. ; S LEININGER ; C SCHLEPER ; JI. PROSSER.** The influence of soil pH on the diversity, abundance and transcriptional activity of ammonia oxidizing archaea and bacteria. *Environ. Microbiol.,* 2008, vol. 10, 2966-2978
- **OWNLEY BH ; DM WELLER ; LS THOMASHOW.** Influence of in situ and in vitro pH on suppression of Gaeumannomyces graminis var. tritici by Pseudomonas fluorescens 2-79. *Phytopathology,* 1992, vol. 82, 178-184
- **PANKRATOV, TA ; YM SERKEBAEVA ; IS KULICHEVSKAYA ; W LIESACK ; SV DEDYSH ; 2008.** Substrate inudced growth and isolation of Acidobacteria from acidic Sphagnum peat. *The ISME J.,* vol. 2, 551-560
- **PARKIN, TB. ; AJ. SEXSTONE ; JM. TIEDJE.** Soil pH Adaptation of Denitrifying Populations to Low. *Appl. Environ. Microbiol. 1985,* 1985, vol. 49 (5), 1053
- **PAUTLER, MC ; JTH SIMS.** Relationships Between Soil Test Phosphorus, Soluble Phosphorus, and Phosphorus Saturation in Delaware Soils. *Soil Sci. Soc. Am. J.,* 2000, vol. 64, 765-773
- **PETERSE, F ; GW. NICOL ; S SCHOUTEN ; JS SINNINGHE DAMSTÉ.** Influence of soil pH on the abundance and distribution of core and intact polar lipid-derived branched GDGTs in soil. *Organic. Geochem.,* 2010, vol. 41, 1171-75
- **PICARD, C ; C PONSONNET ; E PAGET ; X NESME ; P. SIMONET.** Detectionand Enumeration ofBacteriainSoilby Direct DNA Extractionand Polymerase Chain Reaction. *Appl. Environ. Microbiol,* 1992, vol. 58 (9), 2717-2722
- **ROPER MM ; RR GAULT ; NA SMITH.** Contribution to the N status of soil by free living nitrogen fixing bacteria in a lucerne stand. *Soil Biol. Biochem.,* 1995, vol. 27 (4/5), 467-471
- **ROSSO, L. ; J R LOBRY ; S BAJARD ; J P FLANDROIS.** Effects of Temperature and pH on Microbial Growth. Convenient Model To Describe the Combined. *Appl. Environ. Microbiol.,* 1995, vol. 61 (2), 610
- **ROUSK ; JOHANNES ; DAVEY L. JONES.** Loss of low molecular weight dissolved organic carbon (DOC) and nitrogen (DON) in H2O and 0.5 M K2SO4 soil extracts. *Soil Biol. Biochem.,* 2010, vol. 42, 2331-2335
- **ROUSK, J. ; PHILIP C. BROOKES ; ERLAND BÅÅTH.** The microbial PLFA composition as affected by pH in an arable soil. *Soil Biology & Biochemistry,* 2010, vol. 42, 516-520
- **ROUSK, J. ; PC. BROOKES ; E BÅÅTH.** Investigating the mechanisms for the opposing pH relationships of fungal and bacterial growth in soil. *Soil Biology & Biochemistry,* 2010, vol. 42, 926e934
- **ROUSK, J ; PC. BROOKES ; E BÅÅTH.** Contrasting Soil pH Effects on Fungal and Bacterial Growth Suggest Functional Redundancy in Carbon Mineralization. *Appl. Environ. Microbiol.,* Mars 2009, vol. 75 (6), 1589-1596

- **ROUSK, J. ; PC. BROOKES ; HC. GLANVILLE ; DL. JONES.** Lack of Correlation between Turnover of Low-Molecular-Weight Dissolved Organic Carbon and Differences in Microbial Community Composition or Growth across a Soil pH Gradient. *Appl. Environ. Microbiol.,* 2011, vol. 77 (8), 2791-2795
- **ROUSK, J. ; E BAATH ; PC BROOKES ; CL LAUBER ; C LOZUPONE ; JG CAPORASO ; R KNIGHT ; N FIERER.** Soil bacterial and fungal communities across a pH gradient in an arable soil. *ISME Journal,* 2010, vol. 4, 1340-1351
- **SESSITSCH, A. ; E. HACKL ; P. WENZL ; A. KILIAN ; T. KOSTIC ; N. STRALIS-PAVESE ; B. TANKOUO SANDJONG ; L. BODROSSY.** Diagnostic microbial microarrays in soil ecology. *New Phytologist,* 2006, vol. 171, 719-736
- **IMEK, M. ; D.W. HOPKINS.** Regulation of potential denitrification by soil pH in long-term fertilized arable soils. *Biol Fertil Soils,* 1999, vol. 30, 41-47
- **SIMEK, M. ; L JISOVA ; DW. HOPKINS.** What is the so-called optimum pH for denitrification in soil. *Soil Biol Biochem.,* 2002, vol. 34, 1227-1234
- **TAZIEBOU, LC ; FX ETOA ; JJ ESSIA NGANG.** Effects of some environmental factors on microbial growth and biodegradability of a pesticide mixture by soil microorganimsms. *J. Camer. Acad. Sci.,* 2004, vol. 4, 41-47
- **WAKELIN, SA. ; L.M.MACDONALD ; S.L.ROGERS ; A.L.GREGGA ; T.P.BOLGER ; J.A.BALDOCK.** Habitat selective factors influencing the structural composition and functional capacity of microbial communities in agricultural soils. *Soil Biol. Biochem.,* 2008, vol. 40, 803-813
- Statistique - économie, gestion, sciences, médecine. **WONNACOTT, TH ; RJ WONNACOTT.** Economia. 1991
- **WU, U. ; B MA ; L ZHOU ; H WANG ; J XU ; S KEMMITT ; PC. BROOKES.** Changes in the soil microbial community structure with latitude in eastern China, based on phospholipid fatty acid analysis. *Appl. Soil Ecol.,* vol. 43, 234-240